(19) Europäisches Patentamt
European Patent Office
Office européen des brevets

(11) Veröffentlichungsnummer : **0 275 828 B1**

(12) **EUROPÄISCHE PATENTSCHRIFT**

(45) Veröffentlichungstag der Patentschrift : **20.03.91 Patentblatt 91/12**

(51) Int. Cl.$^5$ : **C07C 335/24,** C07C 335/30, A01N 47/34

(21) Anmeldenummer : **87810750.7**

(22) Anmeldetag : **14.12.87**

# (54) N-Phenyl-N-carboxyl-thioharnstoffe und ihre Verwendung bei der Kontrolle von Schädlingen.

(30) Priorität : **17.12.86 CH 5056/86**
**29.07.87 CH 2909/87**

(43) Veröffentlichungstag der Anmeldung : **27.07.88 Patentblatt 88/30**

(45) Bekanntmachung des Hinweises auf die Patenterteilung : **20.03.91 Patentblatt 91/12**

(84) Benannte Vertragsstaaten : **AT BE CH DE FR GB IT LI NL**

(56) Entgegenhaltungen :
**EP-A- 0 000 365**
**CH-A- 506 505**

(56) Entgegenhaltungen :
**JOURNAL OF MEDICINAL CHEMISTRY, Band 23, Nr. 12, 7. April 1980, Seite 1438, Spalte 2, Zeilen 2-4, Washington, D.C., US; J.W. TILLEY et al.: "Antihypertensive (2-Aminoethyl)thiourea Derivatives. 2"**
**L. TOLDY et al.: "Thiocarbamidderivate mit Tuberkulostatischer Wirkung, II", ACTA CHIMICA ACADEMIAE HUNGARICAE, Band 69(2), 1970, Seiten 221-227, Tabelle 3a,**

(73) Patentinhaber : **CIBA-GEIGY AG**
**Klybeckstrasse 141**
**CH-4002 Basel (CH)**

(72) Erfinder : **Drabek, Josef, Dr.**
**Benkenstrasse 12**
**CH-4104 Oberwil (CH)**
Erfinder : **Ehrenfreund, Josef, Dr.**
**Amselstrasse 11**
**CH-4123 Allschwill (CH)**

**EP 0 275 828 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue substituierte N-Phenyl-N-carboxyl-thioharnstoffe, Verfahren und Zwischenprodukte zu ihrer Herstellung, Schädlingsbekämpfungsmittel, welche diese Verbindungen enthalten, sowie ihre Verwendung in der Schädlingsbekämpfung.

Die erfindungsgemässen Verbindungen entsprechen der Formel I

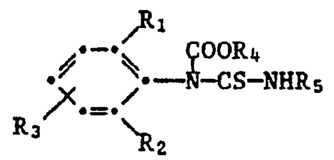

(I),

worin

$R_1$ und $R_2$ je für $C_1$-$C_{10}$-Alkyl, $C_3$-$C_7$-Cycloalkyl oder $C_5$-$C_6$-Cycloalkenyl,

$R_3$ für Wasserstoff, Halogen, $C_1$-$C_{10}$-Alkyl, $C_1$-$C_{10}$-Alkoxy oder $C_1$-$C_{10}$-Alkylthio,

$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle, Alkoxy- und Alkylthioreste können geradkettig oder verzweigt sein. Als Beispiele solcher Alkyle seien u.a. Methyl, Aethyl, Propyl, Isopropyl, Butyl, i-Butyl, sek.Butyl, tert.Butyl oder Pentyl, Hexyl, Octyl und ihre Isomeren genannt. Als geeignete Alkoxy- und Alkylthioreste seien u.a. genannt : Methoxy, Methylthio, Aethoxy, Aethylthio, Propoxy, Propylthio, Isopropoxy, Isopropylthio oder Butoxy und Butylthio und ihre Isomeren.

Die als Substituenten in Betracht kommenden Alkenyle und Alkinyle können geradkettig oder verzweigt sein und eine oder mehrere ungesättigte Bindungen enthalten. Dabei ist es auch möglich, dass zwei- und dreifach ungesättigte Bindungen im gleichen Rest auftreten können. Zweckmässigerweise enthalten diese ungesättigten Reste ein bis fünf Kohlenstoffatome. Beispiele solcher Alkenyle und Alkinyle sind u.a. Vinyl, Allyl, 1-Propenyl, Isopropenyl, Allenyl, Butenyle, Butadienyle, Hexenyle, Hexandienyl, Aethinyl, 1-Propinyl, 2-Propinyl, Butinyle, Pentinyle, Hexinyle, Hexadiinyle, 2-Penten-4-inyl.

Die als Substituenten in Betracht kommenden Phenylalkyle können geradkettig oder verzweigt sein. Geeignete Beispiele sind u.a. Benzyl, Phenäthyl, Phenpropyl, Phenisopropyl, Phenbutyl und seine Isomeren.

Sind die als Substituenten in Betracht kommenden Alkyle, Alkenyle, Alkinyle oder Phenylalkyle durch Halogen substituiert, so können sie nur teilweise oder auch perhalogeniert sein. Die Halogensubstitution der Phenylalkyle kann sowohl im Phenylkern als auch in der Alkylkette oder in beiden gleichzeitig erfolgen. Dabei gelten für die Halogene, Alkyle, Alkenyle, Alkinyle und Phenylalkyle die oben gegebenen Definitionen. Beispiele solcher Gruppen sind u.a. das ein- bis dreifach durch Fluor, Chlor und/oder Brom substituierte Methyl wie z.B. $CHF_2$ oder $CF_3$ ; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$ ; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$ ; das ein-bis neunfach durch Fluor, Chlor und/oder Brom substituierte Butyl oder eines seiner Isomeren wie z.B. $CF(CF_3)CHFCF_3$ oder $CH_2(CF_2)_2CF_3$ ; das ein- bis dreifach durch Fluor, Chlor und/oder Brom substituierte Vinyl, Propinyl oder Pentadiinyl ; das ein- bis fünffach durch Fluor, Chlor und/oder Brom substituierte Allyl, 1-Propenyl, Butadienyl oder ein Butinyl ; ein ein- bis siebenfach durch Fluor, Chlor und/oder Brom substituiertes Butenyl, Pentadienyl oder Pentinyl ; das ein- bis siebenfach durch Fluor, Chlor und/oder Brom substituierte Benzyl ; das ein- bis neunfach durch Fluor, Chlor und/oder Brom substituierte Phenäthyl ; das ein- bis elffach durch Fluor, Chlor und/oder Brom substituierte Phenpropyl oder Phenisopropyl.

Bei den als Substituenten in Betracht kommenden Alkylen, Alkenylen, Alkinylen und Phenylalkylen, die durch Sauerstoff und/oder Schwefel unterbrochen sind, kann es sich sowohl um einfache Aether als auch um Polyalkylenglykole oder -thioglykole handeln. Dabei sind auch Ketten möglich, die Sauerstoff und Schwefel

gleichzeitig enthalten. Beispiele sind u.a. Reste wie Methoxymethyl, Methylthiomethyl, Methoxymethoxymethyl, Methoxyäthyl, Aethoxyäthyl, Aethylthioäthyl, Vinyloxymethyl, Allylthioäthyl, Methoxypropinyl, Benzyloxymethyl, Benzylthioäthyl.

Bei den als Substituenten in Betracht kommenden Cycloalkylen und Cycloalkenylen handelt es sich beispielsweise um Cyclopropyl, Cyclobutyl, Cyclopentyl, 2-Cyclopenten-l-yl, Cyclohexyl oder Cyclohexenyl. Diese Reste können ein- oder mehrfach durch Halogen oder Alkyl substituiert sein wie z.B. 2-Chlorcyclopropyl, 2,2-Dimethylcyclopropyl, 3-Chlorcyclobutyl, 2-Methylcyclohexyl, 2,4-Dimethylcyclohexyl, 2,4,6-Trimethylcyclohexyl.

Unter den Verbindungen der Formel I stehen diejenigen in Vordergrund, in denen

$R_1$ für $C_1$-$C_5$-Alkyl, Cyclopentyl oder Cyclopentenyl,

$R_2$ für $C_1$-$C_5$-Alkyl, oder

$R_1$ und $R_2$ je für $C_1$-$C_6$-Alkyl,

$R_3$ für Wasserstoff, Halogen, $C_1$-$C_3$-Alkyl oder $C_1$-$C_3$-Alkoxy,

$R_4$ für $C_1$-$C_5$-Alkyl, $C_2$-$C_5$-Alkenyl, $C_2$-$C_5$-Alkinyl ; ein- oder mehrfach durch Halogen substituiertes oder durch Sauerstoff und/oder Schwefel unterbrochenes $C_1$-$C_6$-Alkyl, $C_2$-$C_5$-Alkenyl, $C_2$-$C_6$-Alkinyl oder Phenyl-$C_1$-$C_5$-alkyl ; oder $C_3$-$C_6$-Cycloalkyl und

$R_5$ für $C_1$-$C_5$-Alkyl oder ein- oder mehrfach durch Halogen substituiertes oder durch Sauerstoff und/oder Schwefel unterbrochenes $C_1$-$C_5$-Alkyl stehen.

Dabei sind diejenigen Verbindungen der Formel I bevorzugt, in denen

$R_1$ für $C_1$-$C_3$-Alkyl oder Cyclopentyl,

$R_2$ für $C_1$-$C_3$-Alkyl,

$R_3$ für Wasserstoff und

$R_4$ und $R_5$ für $C_1$-$C_4$-Alkyl stehen.

Beispiele von Verbindungen der Formel I sind u.a.

| $R_1$ | $R_2$ | $R_3$ | $R_4$ | $R_5$ |
|---|---|---|---|---|
| $CH_3$ | $CH_3$ | H | $CH_3$ | $CH_3$ |
| $CH_3$ | $CH_3$ | 4-$CH_3$ | $CH_3$ | $CH_3$ |
| $CH_3$ | $CH_3$ | H | $CH_3$ | $C_2H_5$ |
| $CH_3$ | $CH_3$ | H | $C_2H_5$ | $C_2H_5$ |
| $CH_3$ | $CH_3$ | H | $C_2H_5$ | $CH(CH_3)_2$ |
| $CH_3$ | $CH(CH_3)_2$ | H | $CH_3$ | $CH(CH_3)_2$ |
| $CH_3$ | $C_2H_5$ | 4-$SCH_3$ | $C_2H_5$ | $(CH_2)_2(CH_3)$ |
| $C_2H_5$ | $CH_3$ | H | $CH_3$ | $CH_3$ |
| $C_2H_5$ | $CH_3$ | H | $C_2H_5$ | $C_2H_5$ |
| $C_2H_5$ | $CH_3$ | H | $CH(CH_3)_2$ | $CH(CH_3)_2$ |
| $C_2H_5$ | $C_2H_5$ | H | $CH_3$ | $CH_3$ |
| $C_2H_5$ | $C_2H_5$ | H | $C_2H_5$ | $C_2H_5$ |
| $C_2H_5$ | $C_2H_5$ | H | $CH_3$ | $CH(CH_3)_2$ |
| $C_2H_5$ | $C_2H_5$ | H | $CH_3$ | $C(CH_3)_3$ |
| $C_2H_5$ | $C_2H_5$ | H | $(CH_2)_3CH_3$ | $CH(CH_3)_2$ |
| $C_2H_5$ | $C_2H_5$ | H | $CH_2CH_2Cl$ | $CH(CH_3)_2$ |
| $C_2H_5$ | $C_2H_5$ | H | $CH_2-C_6H_5$ | $CH(CH_3)_2$ |
| $C_2H_5$ | $C_2H_5$ | H | $CH_3$ | Cyclopentyl |
| $C_2H_5$ | $C_2H_5$ | 4-$O(CH_2)_3CH_3$ | $C_2H_5$ | $CH(CH_3)_2$ |
| $C_2H_5$ | $CH(CH_3)C_2H_5$ | H | $C_2H_5$ | $CH(CH_3)_2$ |
| $(CH_2)_2CH_3$ | $CH_3$ | H | $CH_3$ | $CH_3$ |
| $(CH_2)_2CH_3$ | $C_2H_5$ | H | $C_2H_5$ | $C_2H_5$ |
| $(CH_2)_2CH_3$ | $(CH_2)_2CH_3$ | H | $(CH_2)_2CH_3$ | $(CH_2)_2CH_3$ |
| $(CH_2)_2CH_3$ | $CH(CH_3)_2$ | H | $CH(CH_3)_2$ | $CH(CH_3)_2$ |
| $CH(CH_3)_2$ | $CH_3$ | H | $CH_3$ | $CH_3$ |
| $CH(CH_3)_2$ | $C_2H_5$ | H | $C_2H_5$ | $C_2H_5$ |
| $CH(CH_3)_2$ | $C_2H_5$ | H | $C_2H_5$ | $CH(CH_3)_2$ |
| $CH(CH_3)_2$ | $CH(CH_3)_2$ | H | $C_2H_5$ | $(CH_2)_2CH_3$ |
| $CH(CH_3)_2$ | $(CH_2)_2CH_3$ | H | $(CH_2)_2CH_3$ | $(CH_2)_2CH_3$ |
| $CH(CH_3)_2$ | $CH(CH_3)_2$ | 4-Cl | $CH_3$ | $CH_3$ |

| $R_1$ | $R_2$ | $R_3$ | $R_4$ | $R_5$ |
|---|---|---|---|---|
| $CH(CH_3)_2$ | $CH(CH_3)_2$ | H | $CH_3$ | $CH_2CH(CH_3)_2$ |
| $CH(CH_3)_2$ | $CH(CH_3)_2$ | H | $C_2H_5$ | $C_2H_5$ |
| $CH(CH_3)_2$ | $CH(CH_3)_2$ | H | $(CH_2)_2CH_3$ | $(CH_2)_2CH_3$ |
| $CH(CH_3)_2$ | $CH(CH_3)_2$ | H | $CH(CH_3)_2$ | $CH(CH_3)_2$ |
| $CH(CH_3)_2$ | $CH(CH_3)_2$ | H | $(CH_2)_3CH_3$ | $CH(CH_3)_2$ |
| $CH(CH_3)_2$ | $CH(CH_3)_2$ | H | $CH_2CH(CH_3)_2$ | $CH(CH_3)_2$ |
| $CH(CH_3)_2$ | $CH(CH_3)_2$ | H | $(CH_2)_2OCH_3$ | $CH(CH_3)_2$ |
| $CH(CH_3)_2$ | $CH(CH_3)_2$ | H | $C_2H_5$ | $CH(CH_3)C_2H_5$ |
| $CH(CH_3)_2$ | $CH(CH_3)_2$ | H | $CH_2-C_6H_5$ | $CH(CH_3)_2$ |
| $CH(CH_3)_2$ | $CH(CH_3)_2$ | H | $(CH_2)_3CH_3$ | $CH(CH_3)_2$ |
| $CH(CH_3)_2$ | $CH(CH_3)_2$ | H | $(CH_2)_2Cl$ | $CH(CH_3)_2$ |
| $CH(CH_3)_2$ | $CH(CH_3)_2$ | H | $CH=CH_2$ | $CH(CH_3)_2$ |
| $CH(CH_3)_2$ | $CH(CH_3)_2$ | H | $(CH_2)_2CCl_3$ | $CH(CH_3)_2$ |
| $CH(CH_3)_2$ | $CH(CH_3)_2$ | H | $CH_3$ | $CH(CH_3)CH_2OCH_3$ |
| $CH(CH_3)_2$ | $CH(CH_3)_2$ | H | $CH_3$ | $CH_2C(CH_3)_2SCH_3$ |
| $CH(CH_3)_2$ | $CH(CH_3)_2$ | H | $C(CH_3)_3$ | $CH(CH_3)_2$ |
| $CH(CH_3)_2$ | $CH(CH_3)_2$ | 4-Br | $CH_3$ | $CH(CH_3)_2$ |
| $CH(CH_3)_2$ | $CH(CH_3)_2$ | 4-Br | $C_2H_5$ | $CH(CH_3)_2$ |
| $CH(CH_3)_2$ | $CH(CH_3)_2$ | $4-OCH_3$ | $CH_3$ | $CH(CH_3)_2$ |
| $C_2H_5$ | $CH(CH_3)_2$ | $4-OC_2H_5$ | $CH_3$ | $CH(CH_3)_2$ |
| $C_2H_5$ | $CH(CH_3)_2$ | $4-OC_2H_5$ | $C_2H_5$ | $CH(CH_3)_2$ |
| $C_2H_5$ | $CH(CH_3)_2$ | $4-CH_3$ | $CH_3$ | $CH(CH_3)_2$ |
| $C_2H_5$ | $CH(CH_3)_2$ | $4-CH_3$ | $C_2H_5$ | $CH(CH_3)_2$ |
| $CH(CH_3)_2$ | $CH(CH_3)_2$ | $4-CH_3$ | $CH_3$ | $CH(CH_3)_2$ |
| $CH(CH_3)_2$ | $CH(CH_3)_2$ | $4-CH_3$ | $C_2H_5$ | $CH(CH_3)_2$ |
| $CH(CH_3)_2$ | $CH(CH_3)_2$ | $4-C_2H_5$ | $CH_3$ | $CH(CH_3)_2$ |
| $CH(CH_3)_2$ | $CH(CH_3)_2$ | $4-C_2H_5$ | $C_2H_5$ | $CH(CH_3)_2$ |
| $CH(CH_3)_2$ | $CH(CH_3)_2$ | $4-CH(CH_3)_2$ | $CH_3$ | $CH(CH_3)_2$ |
| $CH(CH_3)_2$ | $CH(CH_3)_2$ | $4-CH(CH_3)_2$ | $C_2H_5$ | $CH(CH_3)_2$ |

Die erfindungsgemässen Verbindungen der Formel I können nach im Prinzip bekannten Methoden hergestellt werden, indem man z.B.

a) einen Thioharnstoff der Formel II

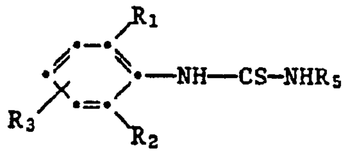

(II)

mit einem Halogenameisensäureester der Formel III

$XCOOR_4$ (III)

in einem Lösungsmittel und in Gegenwart einer Base unter normalem Druck bei – 30 bis + 100°C umsetzt oder

b) ein Salz eines Isothioharnstoffes der Formel IV

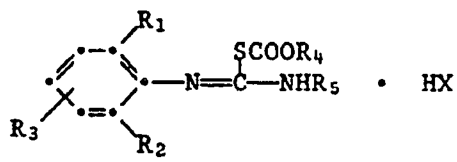

(IV)

bei Raumtemperatur in einer wässrigen Lösung hydrolisiert. In den Formeln II bis IV haben $R_1$, $R_2$, $R_3$, $R_4$ und $R_5$ die für Formel I angegebene Bedeutung und X steht für den Rest einer Halogenwasserstoffsäure, insbesondere für Chlor.

Als Lösungsmittel eignen sich gegenüber den Reaktionsteilnehmern inerte Lösungs- und Verdünnungsmittel wie z.B. Aether oder ätherartige Verbindungen wie u.a. Diäthyläther, Di-isopropyläther, Dioxan oder Tetrahydrofuran ; aromatische Kohlenwasserstoffe wie Benzol, Toluol oder Xylole ; Ketone wie Aceton, Methyläthylketon oder Cyclohexanon ; Nitrile wie Acetonitril ; oder Halogenkohlenwasserstoffe wie Chloroform oder Methylenchlorid.

Geeignete Basen können organischen oder anorganischen Ursprungs sein wie z.B. Natriumhydrid, Natrium- oder Calciumcarbonat, tertiäre Amine wie Triäthylamin, Triäthylendiamin oder 4-Dimethylaminopyridin oder Pyridin.

Die Thioharnstoffe der Formell II ihrerseits können nach im Prinzip bekannten Verfahren hergestellt werden, z.B. indem man ein Isothiocyanat der Formel V

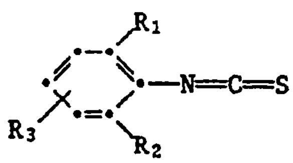

(V)

mit einem Amin der Formel VI

$H_2N\text{-}R_5$ (VI)

umsetzt, wobei in den Formeln V und VI $R_1$, $R_2$, $R_3$ und $R_5$ die für Formel I angegebenen Bedeutungen haben.

Das Verfahren zur Herstellung der Verbindungen der Formel II wird zweckmässigerweise in Gegenwart eines gegenüber den Reaktionsteilnehmern inerten Lösungs- oder Verdünnungsmittels, bei einer Reaktionstemperatur von 0 bis + 100°C unter normalem Druck durchgeführt. Geeignete Lösungs- und Verdünnungsmittel sind beispielsweise schon für das Verfahren zur Herstellung der Verbindungen der Formel I aufgezählt worden.

Die Salze der Isothioharnstoffe der Formel IV können nach im Prinzip bekannten Verfahren hergestellt werden, z.B. indem man einen Thioharnstoff der Formel II mit einem Halogenameisensäureester der Formel III in

einem Lösungsmittel unter normalem Druck bei einer Temperatur von – 20° bis + 100°C umsetzt. Dabei ist die Umsetzung mit Chlorameisensäureester besonders bevorzugt.

Die Verbindungen der Formel IV sind neu und bilden ebenfalls einen Gegenstand der vorliegenden Erfindung. Die Verbindungen der Formeln II, III, V und VI dagegen sind bekannt und können nach im Prinzip bekannten Verfahren hergestellt werden.

Ueberraschenderweise wurde gefunden, dass die erfindungsgemässen Verbindungen der Formel I, sowie die Zwischenprodukte der Formel IV bei günstiger Warmblüter-, Fisch- und Pflanzenverträglichkeit wertvolle Wirkstoffe in der Schädlingsbekämpfung sind. So eignen sich die Verbindungen der Formeln I und IV z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können Larven und Eier von phytopathogenen Schadinsekten und -milben in Zier- und Nutzpflanzungen, wie z.B. in Obst- und Gemüsepflanzungen, aber auch saugende Insekten, wie z.B. Reiszikaden und Aphiden, und Bodeninsekten, wie z.B. Diabrotica-Arten in Maiskulturen, wirkungsvoll bekämpft werden. Werden Verbindungen der Formeln I und IV von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht. Ausserdem weisen die Verbindungen der Formel I und ihre Zwischenprodukte der Formel IV eine beachtliche mikrobizide Wirkung auf. Dabei können vor allem pflanzenpathogene Pilze der Klasse Oomycetidae, wie z.B. Familien der Ordnung Peronosporales (Plasmopara sp.) nachhaltig unter Kontrolle gebracht werden.

Die gute pestizide Wirkung der erfindungsgemässen Verbindungen der Formeln I und IV entspricht einer Abtötungsrate (Mortalität) von mindestens 50-60% der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht : Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formeln I und IV werden in unveränderter Form oder vorzugsweise zusammen mit den in der Formulierungstechnik üblichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formeln I und/oder IV, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyl-äther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formeln I oder IV oder der Kombinationen dieser Wirkstoffe mit andern Insektiziden oder Akariziden nichtionogene, ka-

tion- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bist 22 C-Atomen enthalten und als weitere Substituenten niedrige, gegebenenfalls halogenierte Alkyl-, Benzyl- oder niedrige Hydroxyalkylrest aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979 ; Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95%, Wirkstoff der Formel I oder IV oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1 : Herstellung

1.1. Zwischenprodukte

1.1.1. N-(2,6-Diisopropylphenyl)-N'-sek.butyl-S-methoxycarbonylisothioharnstoff Hydrochlorid

8,8 g N-(2,6-Diisopropylphenyl)-N'-sek.butyl-thioharnstoff werden in 50 ml Aceton gelöst und unter Rühren mit 3,78 g Chlorameisensäuremethylester versetzt. Das Reaktionsgemisch wird 3 Stunden lang weitergerührt und anschliessend 10 Stunden lang bei Raumtemperatur stehen gelassen. Das auskristallisierte Produkt wird abgenutscht und getrocknet. Die Titelverbindung der Formel

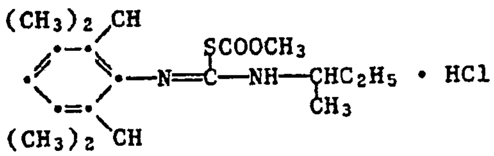

(Verb. 1.1.1.)

liegt in Form farbloser Kristalle vor ; Smp 136-138°C unter Zersetzung.

Auf analoge Weise werden die folgenden Verbindungen hergestellt :

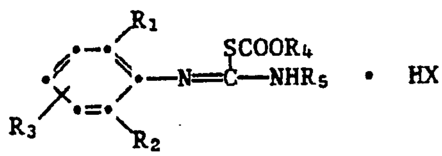

| Verb.Nr. | $R_1$ | $R_2$ | $R_3$ | $R_4$ | $R_5$ | X | Smp.°C |
|---|---|---|---|---|---|---|---|
| 1.1.2. | $CH_3$ | $i\text{-}C_3H_7$ | H | $CH_3$ | $i\text{-}C_3H_7$ | Cl | 120 u.Z. |
| 1.1.3. | $C_2H_5$ | $i\text{-}C_3H_7$ | H | $CH_3$ | $i\text{-}C_3H_7$ | Cl | 124 u.Z. |
| 1.1.4. | $C_2H_5$ | $i\text{-}C_3H_7$ | H | $C_2H_5$ | $i\text{-}C_3H_7$ | Cl | 129 u.Z. |
| 1.1.5. | $C_2H_5$ | $i\text{-}C_3H_7$ | H | $i\text{-}C_4H_9$ | $i\text{-}C_3H_7$ | Cl | 102-103 |
| 1.1.6. | $i\text{-}C_3H_7$ | $i\text{-}C_3H_7$ | H | $CH_3$ | $i\text{-}C_3H_7$ | Cl | 130 u.Z. |
| 1.1.7. | $i\text{-}C_3H_7$ | $i\text{-}C_3H_7$ | H | $C_2H_5$ | $i\text{-}C_3H_7$ | Cl | 141 u.Z. |
| 1.1.8. | $i\text{-}C_3H_7$ | $i\text{-}C_3H_7$ | H | $C_2H_5$ | sek.$C_4H_9$ | Cl | 130 u.Z. |
| 1.1.9. | $CH_3$ | $CH_3$ | H | $CH_3$ | $i\text{-}C_3H_7$ | Cl | 92 u.Z. |
| 1.1.10. | $CH_3$ | $CH_3$ | H | $C_2H_5$ | $i\text{-}C_3H_7$ | Cl | 88-90 u.Z. |
| 1.1.11. | $C_2H_5$ | $C_2H_5$ | H | $C_2H_5$ | $i\text{-}C_3H_7$ | Cl | 126 u.Z. |

| Verb.Nr. | $R_1$ | $R_2$ | $R_3$ | $R_4$ | $R_5$ | X | Smp.°C |
|---|---|---|---|---|---|---|---|
| 1.1.12. | Cyclo-pentyl | i-$C_3H_7$ | H | $CH_3$ | i-$C_3H_7$ | Cl | 166-168 |
| 1.1.13. | Cyclo-pentyl | i-$C_3H_7$ | H | $C_2H_5$ | i-$C_3H_7$ | Cl | 123-124,5 |
| 1.1.14. | Cyclo-pentyl | $C_2H_5$ | H | $C_2H_5$ | i-$C_3H_7$ | Cl | 112-113,5 |
| 1.1.15. | Cyclo-pentyl | $C_2H_5$ | H | $CH_3$ | i-$C_3H_7$ | Cl | 125-127 |
| 1.1.16. | 2-Cyclo-penten-1-yl | $C_2H_5$ | H | $CH_3$ | i-$C_3H_7$ | Cl | 112-113 |
| 1.1.17. | 2-Cyclo-penten-1-yl | i-$C_3H_7$ | H | $CH_3$ | i-$C_3H_7$ | Cl | 162-164,5 |
| 1.1.18. | 2-Cyclo-penten-1-yl | i-$C_3H_7$ | H | $C_2H_5$ | i-$C_3H_7$ | Cl | 127,5-129 |
| 1.1.19. | Cyclo-pentyl | i-$C_3H_7$ | H | $CH_3$ | sek.$C_4H_9$ | Cl | 155-156,5 |
| 1.1.20. | Cyclo-pentyl | i-$C_3H_7$ | H | $C_2H_5$ | sek.$C_4H_9$ | Cl | 124,5-125,5 |
| 1.1.21. | Cyclo-pentyl | $CH_3$ | H | $CH_3$ | i-$C_3H_7$ | Cl | 115,5-117 |
| 1.1.22. | Cyclo-pentyl | $CH_3$ | H | $C_2H_5$ | i-$C_3H_7$ | Cl | 103-104,5 |

1.2. Endprodukte

1.2.1. N-(2,6-Diisopropylphenyl)-N-methoxycarbonyl-N'-isopropylthioharnstoff

15 g N-(2,6-Diisopropylphenyl)-N'-isopropyl-S-methoxycarbonyl-isothioharnstoff Hydrochlorid werden in 250 ml Aethanol gelöst und mit 40 ml Wasser versetzt. Das Reaktionsgemisch wird 12 Stunden lang bei Raumtemperatur stehen gelassen, worauf die entstandenen Kristalle abgenutscht und getrocknet werden. Die Titelverbindung der Formel

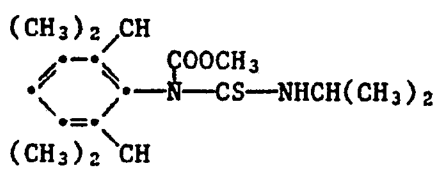

(Verb. 1.2.1.)

liegt in Form farbloser Kristalle vor ; Smp 98-99°C.

Auf analoge Weise werden die folgenden Verbindungen hergestellt :

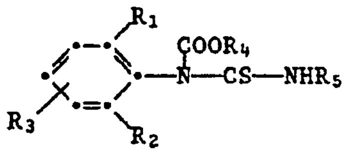

| Verb.Nr. | $R_1$ | $R_2$ | $R_3$ | $R_4$ | $R_5$ | phys. Daten |
|---|---|---|---|---|---|---|
| 1.2.2. | $C_2H_5$ | $i-C_3H_7$ | H | $CH_3$ | $i-C_3H_7$ | Smp. 46-49°C |
| 1.2.3. | $C_2H_5$ | $i-C_3H_7$ | H | $C_2H_5$ | $i-C_3H_7$ | $n_D^{22}$: 1,5355 |
| 1.2.4. | $i-C_3H_7$ | $i-C_3H_7$ | H | $C_2H_5$ | $i-C_3H_7$ | Smp.111-114°C |
| 1.2.5. | $i-C_3H_7$ | $i-C_3H_7$ | H | $i-C_4H_9$ | $i-C_3H_7$ | Smp. 85°C |
| 1.2.6. | Cyclo-pentyl | $i-C_3H_7$ | H | $CH_3$ | $i-C_3H_7$ | Smp. 78-80°C |
| 1.2.7. | Cyclo-pentyl | $i-C_3H_7$ | H | $C_2H_5$ | $i-C_3H_7$ | Smp. 90-92°C |
| 1.2.8. | Cyclo-pentyl | $C_2H_5$ | H | $CH_3$ | $i-C_3H_7$ | $n_D^{23}$: 1,5540 |
| 1.2.9. | Cyclo-pentyl | $C_2H_5$ | H | $C_2H_5$ | $i-C_3H_7$ | $n_D^{23}$: 1,5462 |
| 1.2.10. | 2-Cyclo-penten-1-yl | $i-C_3H_7$ | H | $C_2H_5$ | $i-C_3H_7$ | Smp. 81-83°C |
| 1.2.11. | 2-Cyclo-penten-1-yl | $C_2H_5$ | H | $CH_3$ | $i-C_3H_7$ | $n_D^{25}$: 1,5555 |

| Verb.Nr. | $R_1$ | $R_2$ | $R_3$ | $R_4$ | $R_5$ | phys. Daten |
|---|---|---|---|---|---|---|
| 1.2.12. | 2-Cyclopenten-1-yl | $C_2H_5$ | H | $C_2H_5$ | i-$C_3H_7$ | $n_D^{25}$: 1,5520 |
| 1.2.13. | 2-Cyclopenten-1-yl | i-$C_3H_7$ | H | $CH_3$ | i-$C_3H_7$ | Smp. 78-80°C |
| 1.2.14. | Cyclopentyl | i-$C_3H_7$ | H | $CH_3$ | sek.$C_4H_9$ | $n_D^{24}$: 1,5440 |
| 1.2.15. | Cyclopentyl | i-$C_3H_7$ | H | $C_2H_5$ | sek.$C_4H_9$ | $n_D^{24}$: 1,5382 |
| 1.2.16. | Cyclopentyl | $CH_3$ | H | $CH_3$ | i-$C_3H_7$ | $n_D^{23,5}$: 1,5570 |
| 1.2.17. | Cyclopentyl | $CH_3$ | H | $C_2H_5$ | i-$C_3H_7$ | $n_D^{24}$: 1,5491 |

Beispiel 2 : Formulierungen von Wirkstoffen der Formeln I und IV gemäss den Herstellungsbeispielen 1.1 und 1.2.

(% = Gewichtsprozent)

| 2.1. Emulsions-Konzentrate | a) | b) |
|---|---|---|
| Wirkstoff gemäss dem Herstellungsbeispiel 1.1. oder 1.2. | 10 % | 25 % |
| Ca-Dodecylbenzolsulfonat | - | 5 % |
| Ricinusöl-polyäthylenglykoläther (36 Mol AeO) | 25 % | 5 % |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss dem Herstellungsbeispiel 1.1. oder 1.2. | 10 % | 5 % |
| Polyäthylenglykol (MG 400) | 70 % | - |

| | | |
|---|---|---|
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3. Granulate | a) | b) |
|---|---|---|
| Wirkstoff gemäss dem Herstellungsbeispiel 1.1. oder 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Extruder Granulat | |
|---|---|
| Wirkstoff gemäss dem Herstellungsbeispiel 1.1. oder 1.2. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5. Umhüllungs-Granulat | |
|---|---|
| Wirkstoff gemäss dem Herstellungsbeispiel 1.1. oder 1.2. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
| --- | --- | --- | --- | --- |
| Wirkstoff gemäss dem Herstellungsbeispiel 1.1. oder 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7. Spritzpulver | a) | b) | c) |
| --- | --- | --- | --- |
| Wirkstoff gemäss dem Herstellungsbeispiel 1.1. oder 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.8. Suspensions-Konzentrat | |
| --- | --- |
| Wirkstoff gemäss dem Herstellungsbeispiel 1.1. oder 1.2. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-

Konzentrat, aus welchem durch Verdünnen mit Wasser Suspensionen jeder gewünschten Konzentration hergestellt werden können.

Beispiel 3 : Biologische Prüfungen

3.1. Wirkung gegen Lucilia sericata

Zu 9 ml eines Zuchtmediums wird bei 50°C 1 ml einer 0,5% Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.1. und 1.2. zeigen in diesem Test gute Wirkung gegen Lucilia sericata.

3.2. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss den Beispielen 1.1. und 1.2. zeigen gute Wirkung in diesem Test.

3.3. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50% relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispielen 1.1. und 1.2. zeigen gute Wirkung in diesem Test.

3.4. Wirkung gegen pflanzenschädigende Akarinen : Tetranychus urticae (OP-sensible) und Tetranychus cinnabarinus (OP-tolerant)

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt (die Toleranz bezieht sich auf die Verträglichkeit gegenüber Diazinon).

Die so behandelten infestierten Pflanzen werden mit einer Versuchslösung enthaltend 400 ppm der jeweils zu prüfenden Verbindung bis zur Tropfnässe besprüht. Nach 24 Stunden und wiederum nach 7 Tagen werden Imagines und Larven (alle beweglichen Stadien) unter dem Binokular auf lebende und tote Individuen ausgewertet. Man verwendet pro Konzentration und pro Testspezies eine Pflanze. Während des Versuchsverlaufs stehen die Pflanzen in Gewächshauskabinen bei 25°C.

Verbindungen gemäss den Beispielen 1.1 und 1.2. zeigen in diesem Versuch gute Wirkung gegen Tetranychus urticae, und Tetranychus cinnabarinus.

3.5. Kontaktwirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55% relativer Luftfeuchtigkeit und mit einer Belichtungsperiode von 16 Stunden durchgeführt.

Verbindungen gemäss den Beispielen 1.1. und 1.2. zeigen in diesem Test gute Wirkung.

3.6. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55% relativer Luftfeuchtigkeit und mit einer Belichtungsperiode von 16 Stunden durchgeführt.

Verbindungen gemäss den Beispielen 1.1. und 1.2. zeigen in diesem Test gute Wirkung.

3.7. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60% relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.1. und 1.2. zeigen im obigem Test 80-100%ige Wirkung (Mortalität) gegen Nilaparvata lugens.

3.8. Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmeser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven werden hinsichtlich ihrer Mortalität geprüft.

Verbindungen gemäss den Beispielen 1.1. und 1.2. zeigen gute Wirkung im obigen Test.

3.9. Wirkung gegen Zecken in verschiedenen Entwicklungsstadien

Als Testobjekte werden Larven (jeweils ca 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 400 ppm getaucht. Die in Teströhrchen befindlichen Emulsionen werden dann mit Watte aufgenommen und die benetzten Testtiere in den so kontaminierten Röhrchen belassen. Die Auswertung (% - Mortalität) erfolgt für Larven nach 3 Tagen und für Nymphen und Imagines nach 14 Tagen.

Verbindungen gemäss den Beispielen 1.1. und 1.2. zeigen gute Wirkung im obigen Test.

Beispiel 3.10. Wirkung gegen Plasmopara viticola auf Reben Residual-protektive Wirkung

Im 4-5 Blattstadium werden Rebensämlinge mit einer aus Spritzpulver des Wirkstoffes hergestellten Spritzbrühe (0,02% Aktivsubstanz) besprüht. Nach 24 Stunden werden die behandelten Pflanzen mit einer Sporangiensuspension des Pilzes infiziert. Nach einer Inkubation während 6 Tagen bei 95-100% relativer Luftfeuchtigkeit und 20°C wird der Pilzbefall beurteilt.

Verbindungen gemäss den Beispielen 1.1. und 1.2. zeigen gegen Plasmopara gute Wirksamkeit. So reduziert z.B. die Verbindung Nr. 1.2.13. den Plasmopara-Befall auf 0 bis 5%. Unbehandelte aber infizierte Kontrollpflanzen weisen dagegen einen Plasmopara-Befall von 100% auf.

## Ansprüche

1. Verbindungen der Formel I

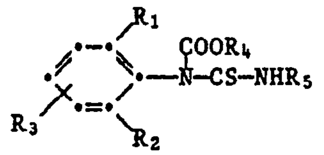

(I),

worin

$R_1$ und $R_2$ je für $C_1$-$C_{10}$-Alkyl, $C_3$-$C_7$-Cycloalkyl oder $C_5$-$C_6$-Cycloalkenyl,
$R_3$ für Wasserstoff, Halogen, $C_1$-$C_{10}$-Alkyl, $C_1$-$C_{10}$-Alkoxy oder $C_1$-$C_{10}$-Alkylthio,
$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und
$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen.

2. Verbindungen der Formel I gemäss Anspruch 1, worin
$R_1$ für $C_1$-$C_5$-Alkyl, Cyclopentyl oder Cyclopentenyl,
$R_2$ für $C_1$-$C_5$-Alkyl,
$R_3$ für Wasserstoff, Halogen, $C_1$-$C_3$-Alkyl oder $C_1$-$C_3$-Alkoxy,
$R_4$ für $C_1$-$C_5$-Alkyl, $C_2$-$C_5$-Alkenyl, $C_2$-$C_5$-Alkinyl ; ein- oder mehrfach durch Halogen substituiertes oder durch Sauerstoff und/oder Schwefel unterbrochenes $C_1$-$C_5$-Alkyl, $C_2$-$C_5$-Alkenyl, $C_2$-$C_5$-Alkinyl oder Phenyl-$C_1$-$C_5$-alkyl ; oder $C_3$-$C_6$-Cycloalkyl und
$R_5$ für $C_1$-$C_5$-Alkyl oder ein- oder mehrfach durch Halogen substituiertes oder durch Sauerstoff und/oder Schwefel unterbrochenes $C_1$-$C_5$-Alkyl stehen.

3. Verbindungen der Formel I gemäss Anspruch 2, worin
$R_1$ für $C_1$-$C_3$-Alkyl oder Cyclopentyl,
$R_2$ für $C_1$-$C_3$-Alkyl,
$R_3$ für Wasserstoff und
$R_4$ und $R_5$ für $C_1$-$C_4$-Alkyl stehen.

4. Verbindungen gemäss Anspruch 2 der Formeln

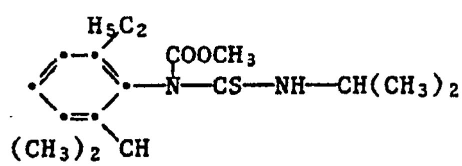

,

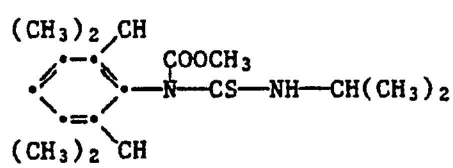

,

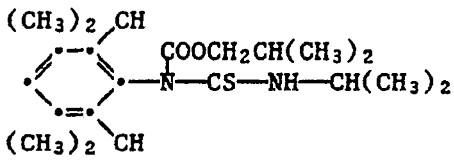

,

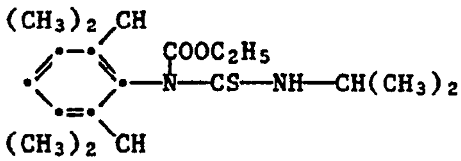

,

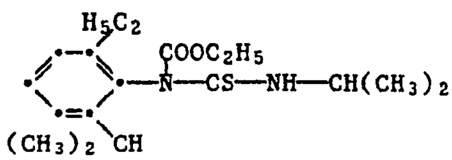

,

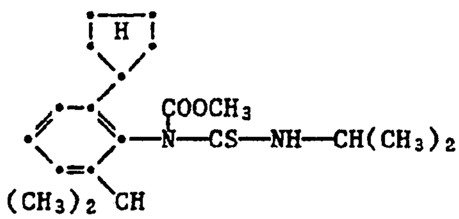

,

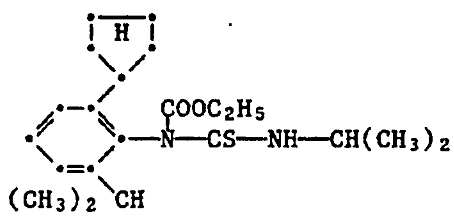

,

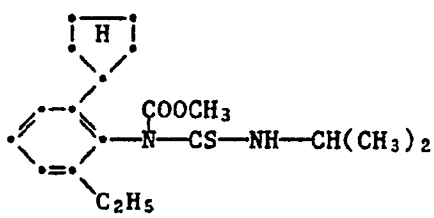

,

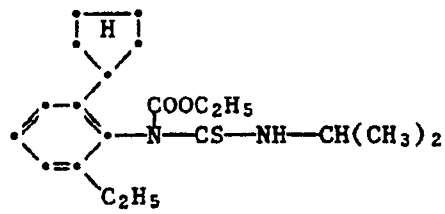

,

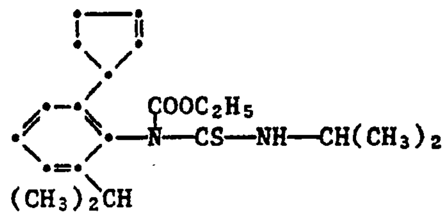

,

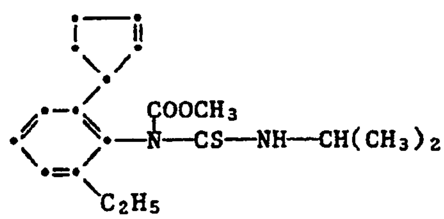

,

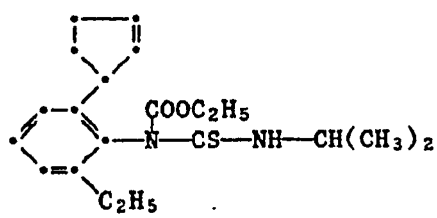

,

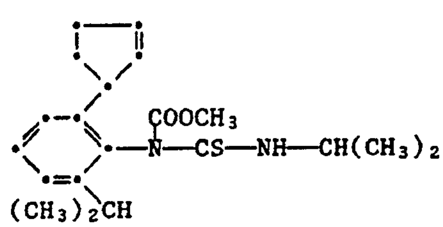

,

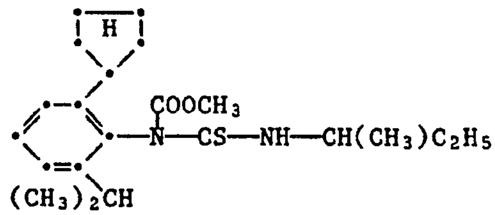

,

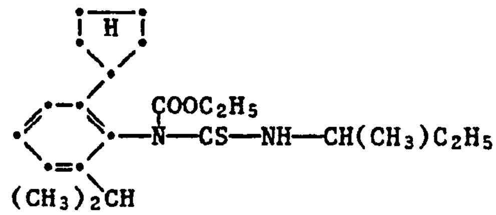

,

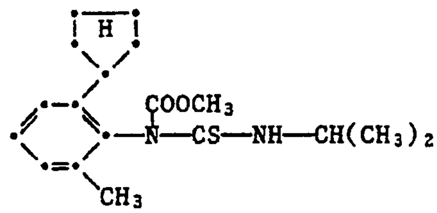

und

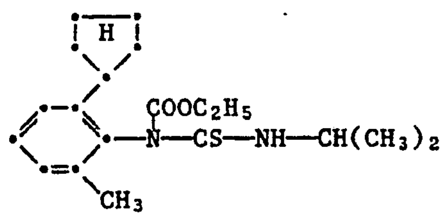

.

5. Verfahren zur Herstellung einer Verbindung der Formel I

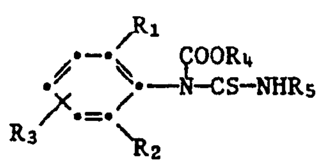

(I),

worin

$R_1$ und $R_2$ je für $C_1$-$C_{10}$-Alkyl, $C_3$-$C_7$-Cycloalkyl oder $C_5$-$C_6$-Cycloalkenyl,

$R_3$ für Wasserstoff, Halogen, $C_1$-$C_{10}$-Alkyl, $C_1$-$C_{10}$-Alkoxy oder $C_1$-$C_{10}$-Alkylthio,

$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder

mehrfach durch Sauerstoff und/oder Schwefel unterbrochenes $C_1$-$C_{10}$-Alkyl oder Phenyl-$C_1$-$C_7$- alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, dadurch gekennzeichnet, dass man
a) einen Thioharnstoff der Formel II

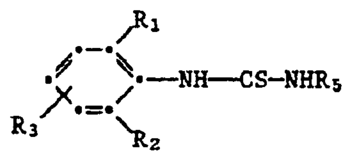

(II)

mit einem Halogenameisensäureester der Formel III

$$XCOOR_4 \quad (III)$$

in einem Lösungsmittel und in Gegenwart einer Base unter normalem Druck bei – 30 bis + 100°C umsetzt oder
b) ein Salz eines Isothioharnstoffs der Formel IV

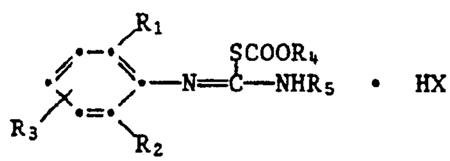

(IV)

bei Raumtemperatur in einer wässrigen Lösung hydrolysiert, wobei in den Formeln II bis IV $R_1$ bis $R_5$ die angegebene Bedeutung haben und X dem Rest einer Halogenwasserstoffsäure entspricht.
6. Schädlingsbekämpfungsmittel, welches als aktive Komponente mindestens eine Verbindung der Formel I

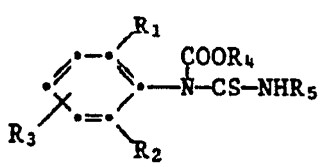

(I)

oder eine Verbindung der Formel IV

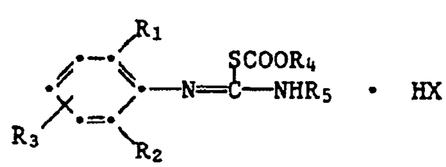

(IV)

enthält, worin
$R_1$ und $R_2$ je für $C_1$-$C_{10}$-Alkyl, $C_3$-$C_7$-Cycloalkyl oder $C_5$-$C_6$-Cycloalkenyl,
$R_3$ für Wasserstoff, Halogen, $C_1$-$C_{10}$-Alkyl, $C_1$-$C_{10}$-Alkoxy oder $C_1$-$C_{10}$-Alkylthio,
$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, und

X den Rest einer Halogenwasserstoffsäure bedeutet, zusammen mit geeigneten Trägern und/oder Zuschlagstoffen.

7. Schädlingsbekämpfungsmittel gemäss Anspruch 6, welches als aktive Verbindung mindestens eine Verbindung der Formel I oder IV enthält, worin

$R_1$ je für $C_1$-$C_5$-Alkyl, Cyclopentyl oder Cyclopentenyl,

$R_2$ für $C_1$-$C_5$-Alkyl,

$R_3$ für Wasserstoff, Halogen, $C_1$-$C_3$-Alkyl oder $C_1$-$C_3$-Alkoxy,

$R_4$ für $C_1$-$C_5$-Alkyl, $C_2$-$C_5$-Alkenyl, $C_2$-$C_5$-Alkinyl ; ein- oder mehrfach durch Halogen substituiertes oder durch Sauerstoff und/oder Schwefel unterbrochenes $C_1$-$C_5$-Alkyl, $C_2$-$C_5$-Alkenyl, $C_2$-$C_5$-Alkinyl oder Phenyl-$C_1$-$C_5$-alkyl ; oder $C_3$-$C_6$-Cycloalkyl und

$R_5$ für $C_1$-$C_5$-Alkyl oder ein- oder mehrfach durch Halogen substituiertes oder durch Sauerstoff und/oder Schwefel unterbrochenes $C_1$-$C_5$-Alkyl stehen, und

X den Rest einer Halogenwasserstoffsäure bedeutet.

8. Schädlingsbekämpfungsmittel gemäss Anspruch 7, welches als aktive Komponente mindestens eine Verbindung der Formel I oder IV enthält, worin

$R_1$ für $C_1$-$C_3$-Alkyl oder Cyclopentyl,

$R_2$ für $C_1$-$C_3$-Alkyl,

$R_3$ für Wasserstoff und

$R_4$ und $R_5$ für $C_1$-$C_4$-Alkyl stehen und

X Chlor bedeutet.

9. Verwendung einer Verbindung der Formel I

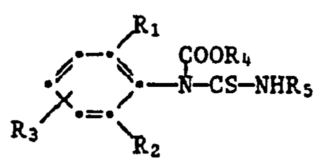

(I)

oder einer Verbindung der Formel IV

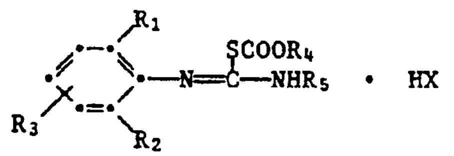

(IV),

worin

$R_1$ und $R_2$ je für $C_1$-$C_{10}$-Alkyl, $C_3$-$C_7$-Cycloalkyl oder $C_5$-$C_6$-Cycloalkenyl,

$R_3$ für Wasserstoff, Halogen, $C_1$-$C_{10}$-Alkyl, $C_1$-$C_{10}$-Alkoxy oder $C_1$-$C_{10}$-Alkylthio,

$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, und

X den Rest einer Halogenwasserstoffsäure bedeutet, zur Bekämpfung von Schädlingen an Tieren und Pflanzen.

10. Verwendung gemäss Anspruch 9 einer Verbindung der Formel I oder IV zur Bekämpfung von Insekten, Arachniden und pflanzenpathogenen Pilzen.

11. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I

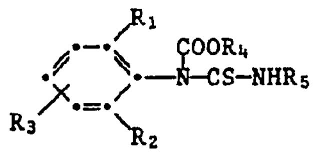

(I)

oder mit einer Verbindung der Formel IV

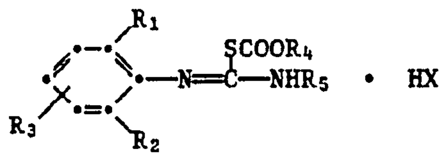

(IV),

worin

$R_1$ und $R_2$ je für $C_1$-$C_{10}$-Alkyl, $C_3$-$C_7$-Cycloalkyl oder $C_5$-$C_6$-Cycloalkenyl,

$R_3$ für Wasserstoff, Halogen, $C_1$-$C_{10}$-Alkyl, $C_1$-$C_{10}$-Alkoxy oder $C_1$-$C_{10}$-Alkylthio,

$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, und

X den Rest einer Halogenwasserstoffsäure bedeutet, in Kontakt bringt.

12. Verbindungen der Formel IV

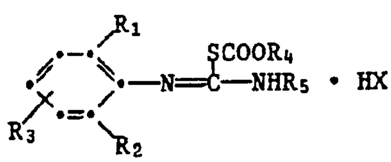

(IV),

worin

$R_1$ und $R_2$ je für $C_1$-$C_{10}$-Alkyl, $C_3$-$C_7$-Cycloalkyl oder $C_5$-$C_6$-Cycloalkenyl,

$R_3$ für Wasserstoff, Halogen, $C_1$-$C_{10}$-Alkyl, $C_1$-$C_{10}$-Alkoxy oder $C_1$-$C_{10}$-Alkylthio,

$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und

$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, und

X den Rest einer Halogenwasserstoffsäure bedeutet.

13. Verbindungen der Formel IV gemäss Anspruch 12, worin

$R_1$ für $C_1$-$C_5$-Alkyl, Cyclopentyl oder Cyclopentenyl,

$R_2$ für $C_1$-$C_5$-Alkyl,

$R_3$ für Wasserstoff, Halogen, $C_1$-$C_3$-Alkyl oder $C_1$-$C_3$-Alkoxy,

$R_4$ für $C_1$-$C_5$-Alkyl, $C_2$-$C_5$-Alkenyl, $C_2$-$C_5$-Alkinyl ; ein- oder mehrfach durch Halogen substituiertes oder

durch Sauerstoff und/oder Schwefel unterbrochenes $C_1$-$C_5$-Alkyl, $C_2$-$C_5$-Alkenyl, $C_2$-$C_5$-Alkinyl oder Phenyl-$C_1$-$C_5$-alkyl ; oder $C_3$-$C_6$-Cycloalkyl und
$R_5$ für $C_1$-$C_5$-Alkyl oder ein- oder mehrfach durch Halogen substituiertes oder durch Sauerstoff und/oder Schwefel unterbrochenes $C_1$-$C_5$-Alkyl stehen und
X den Rest einer Halogenwasserstoffsäure bedeutet.

14. Verbindungen der Formel IV gemäss Anspruch 13, worin
$R_1$ für $C_1$-$C_3$-Alkyl oder Cyclopentyl,
$R_2$ für $C_1$-$C_3$-Alkyl,
$R_3$ für Wasserstoff und
$R_4$ und $R_5$ für $C_1$-$C_4$-Alkyl stehen und
X Chlor bedeutet.

15. Verbindungen gemäss Anspruch 13 der Formeln

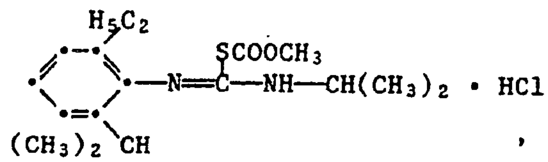

,

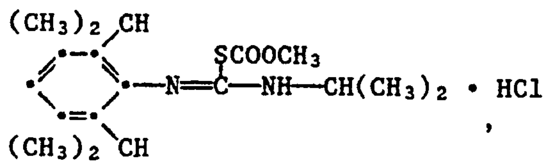

,

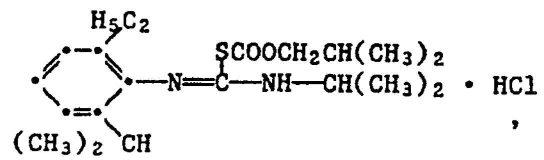

,

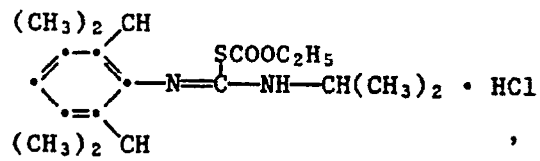

,

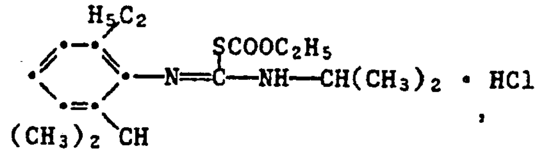

,

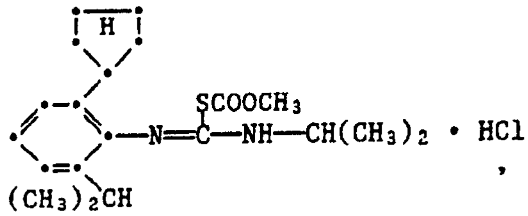

,

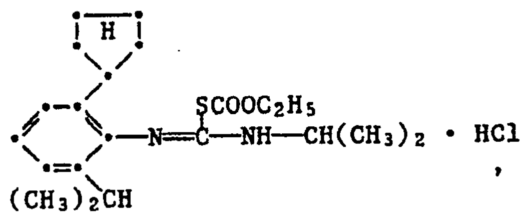

,

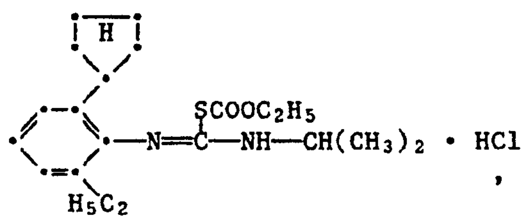

,

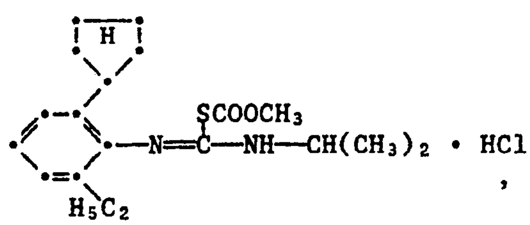

,

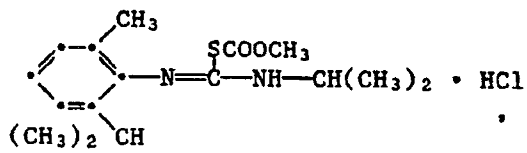

,

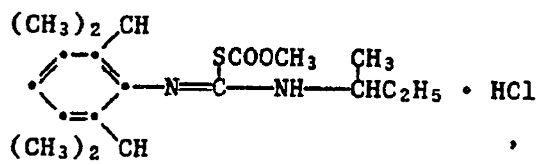

,

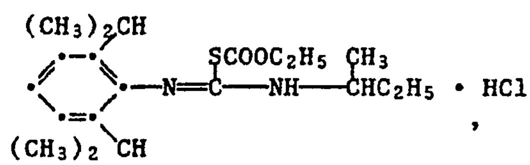

,

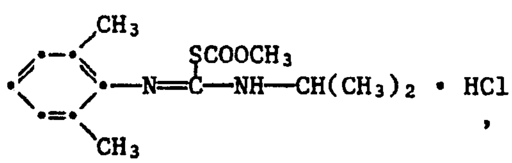

,

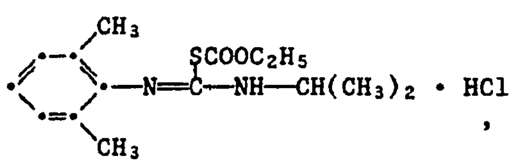

,

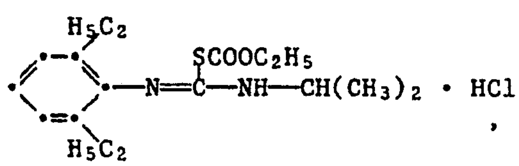

,

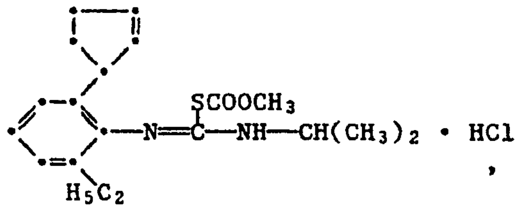

,

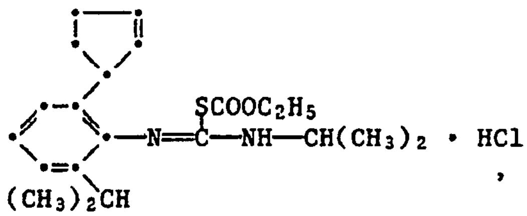

,

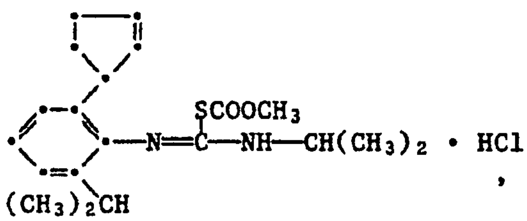

,

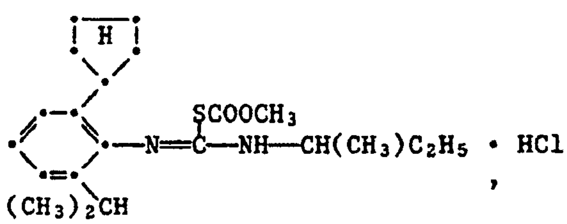

,

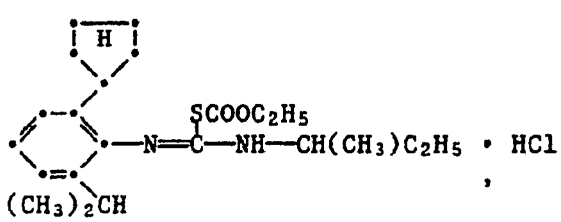

,

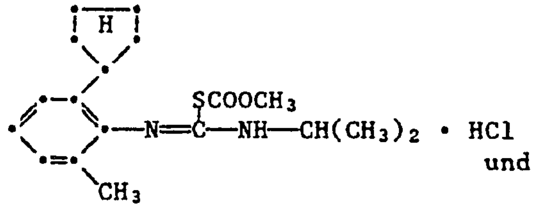

und

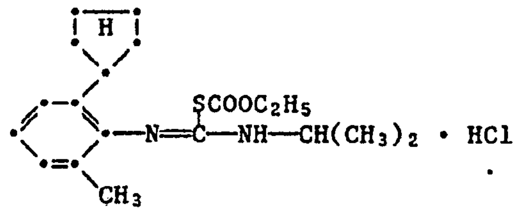

16. Verfahren zur Herstellung einer Verbindung der Formel IV

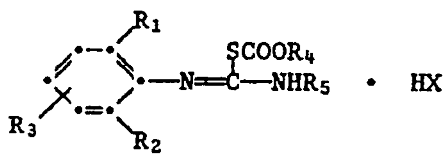

(IV),

worin

$R_1$ und $R_2$ je für $C_1$-$C_{10}$-Alkyl, $C_3$-$C_7$-Cycloalkyl oder $C_5$-$C_6$-Cycloalkenyl,
$R_3$ für Wasserstoff, Halogen, $C_1$-$C_{10}$-Alkyl, $C_1$-$C_{10}$-Alkoxy oder $C_1$-$C_{10}$-Alkylthio,
$R_4$ für $C_1$-$C_{10}$-Alkyl, $C_2$-$C_{10}$-Alkenyl, $C_2$-$C_{10}$-Alkinyl, Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl und
$R_5$ für $C_1$-$C_{10}$-Alkyl ; Phenyl-$C_1$-$C_7$-alkyl ; ein- oder mehrfach durch Halogen substituiertes oder ein- oder mehrfach durch Sauerstoff und/oder Schwefel unterbrochenes $C_1$-$C_{10}$-Alkyl oder Phenyl-$C_1$-$C_7$-alkyl ; $C_3$-$C_8$-Cycloalkyl ; oder ein- oder mehrfach durch Halogen oder $C_1$-$C_5$-Alkyl substituiertes $C_3$-$C_8$-Cycloalkyl stehen, und
X den Rest einer Halogenwasserstoffsäure bedeutet, dadurch gekennzeichnet, dass man einen Thioharnstoff der Formel II

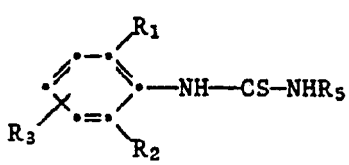

(II)

in einem Lösungsmittel unter normalem Druck bei einer Temperatur von − 20 bis + 100°C mit einem Halogenameisensäureester der Formel III

$$XCOOR_4 \quad (III)$$

umsetzt, wobei $R_1$ bis $R_5$ und X die angegebene Bedeutung haben.

## Claims

1. Compounds of the formula I

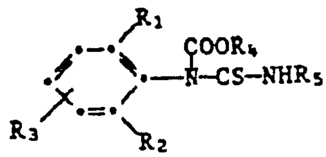

(I),

in which

$R_1$ and $R_2$ each represents $C_1$-$C_{10}$alkyl, $C_3$-$C_7$cycloalkyl or $C_5$-$C_8$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl.

2. Compounds of the formula I according to claim 1, in which

$R_1$ represents $C_1$-$C_5$alkyl, cyclopentyl or cyclopentenyl,

$R_2$ represents $C_1$-$C_5$alkyl,

$R_3$ represents hydrogen, halogen, $C_1$-$C_3$alkyl or $C_1$-$C_3$alkoxy,

$R_4$ represents $C_1$-$C_5$alkyl, $C_2$-$C_5$alkenyl or $C_2$-$C_5$alkynyl ;$C_1$-$C_5$alkyl, $C_2$-$C_5$alkenyl, $C_2$-$C_5$alkynyl or phenyl-$C_1$-$C_5$alkyl each mono- or poly-substituted by halogen or interrupted by oxygen and/or sulphur ; or $C_3$-$C_6$cycloalkyl, and

$R_5$ represents $C_1$-$C_5$alkyl, or $C_1$-$C_5$alkyl mono- or poly-substituted by halogen or interrupted by oxygen and/or sulphur.

3. Compounds of the formula I according to claim 2, in which

$R_1$ represents $C_1$-$C_3$alkyl or cyclopentyl,

$R_2$ represents $C_1$-$C_3$alkyl,

$R_3$ represents hydrogen, and

$R_4$ and $R_5$ represent $C_1$-$C_4$alkyl.

4. Compounds according to claim 2 of the formulae

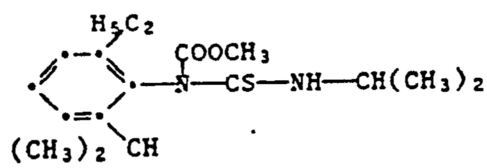

,

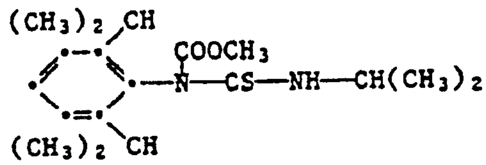

,

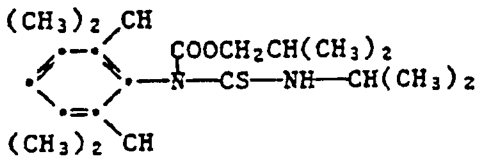

,

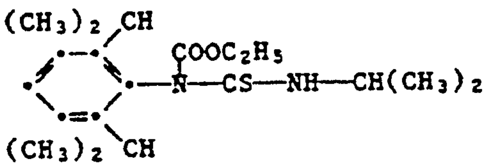

,

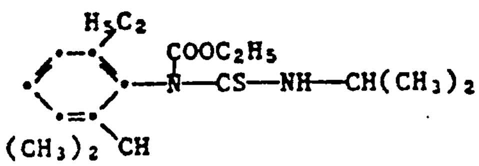

,

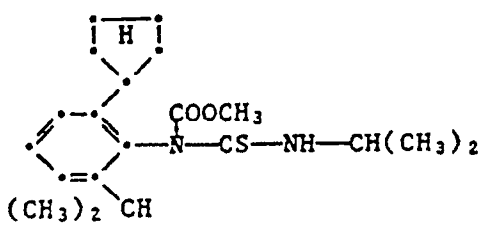

,

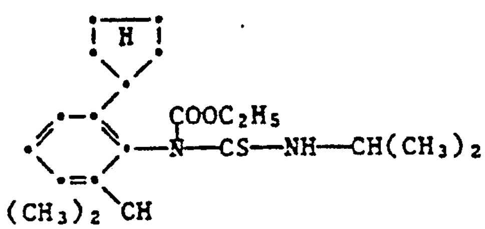

,

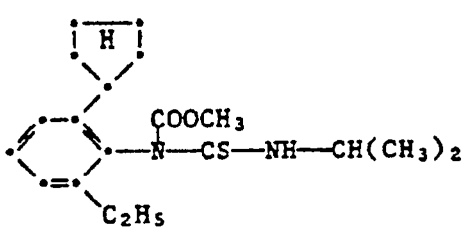

,

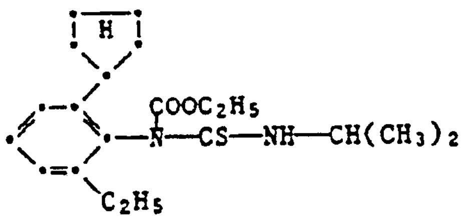

,

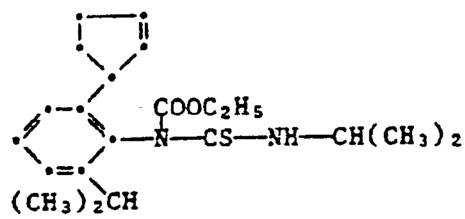

,

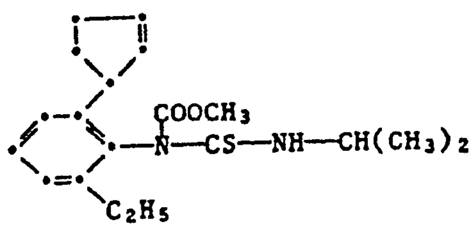

,

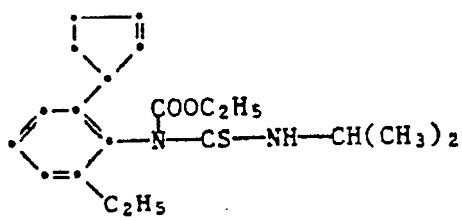

,

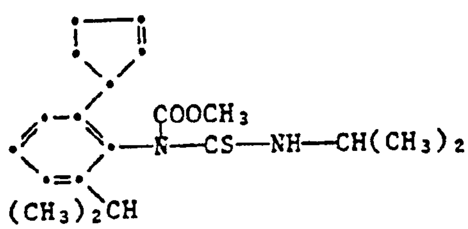

,

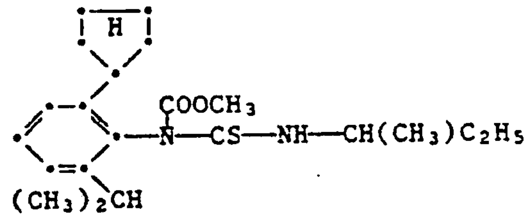

,

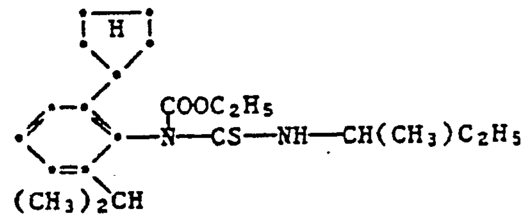

,

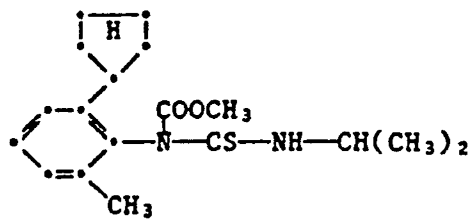

and

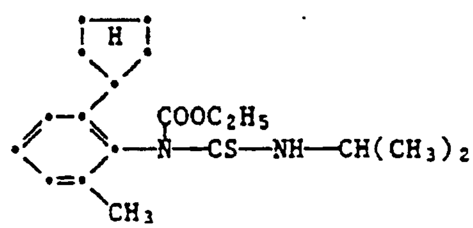

.

5. A process for the preparation of a compound of the formula I

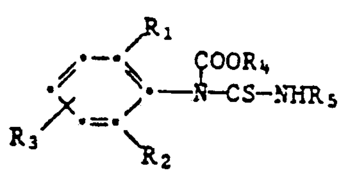

(I),

in which

$R_1$ and $R_2$ each represents $C_1$-$C_{10}$alkyl, $C_3$-$C_7$cycloalkyl or $C_5$-$C_6$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl,

$C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, characterised in that

a) a thiourea of the formula II

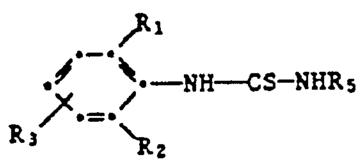

(II)

is reacted with a haloformic acid ester of the formula 111

XCOOR$_4$ (III)

in a solvent and in the presence of a base, under normal pressure and at from − 30 to + 100°C, or

b) a salt of an isothiourea of the formula IV

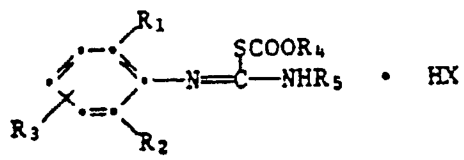

(IV)

is hydrolysed at room temperature in an aqueous solution, in the formulae II to IV $R_1$ to $R_5$ having the meanings indicated and X representing the radical of a hydrohalic acid.

6. A pesticidal composition which contains as active component at least one compound of the formula I

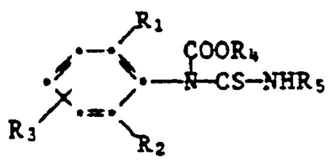

(I)

or one compound of the formula IV

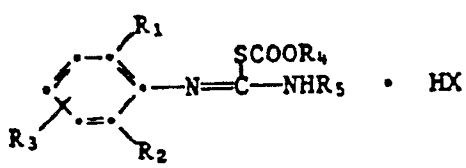

(IV)

in which

$R_1$ and $R_2$ each represents $C_1$-$C_{10}$alkyl, $C_3$-$C_7$cycloalkyl or $C_5$-$C_6$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, and

X represents the radical of a hydrohalic acid, together with suitable carriers and/or adjuvants.

7. A pesticidal composition according to claim 6, which contains as active compound at least one compound of the formula I or IV in which

$R_1$ represents $C_1$-$C_5$alkyl, cyclopentyl or cyclopentenyl,

$R_2$ represents $C_1$-$C_5$alkyl,

$R_3$ represents hydrogen, halogen, $C_1$-$C_3$alkyl or $C_1$-$C_3$alkoxy,

$R_4$ represents $C_1$-$C_5$alkyl, $C_2$-$C_5$alkenyl or $C_2$-$C_5$alkynyl ; $C_1$-$C_5$alkyl, $C_2$-$C_5$alkenyl, $C_2$-$C_5$alkynyl or phenyl-$C_1$-$C_5$alkyl each mono- or poly-substituted by halogen or interrupted by oxygen and/or sulphur ; or $C_3$-$C_6$cycloalkyl, and

$R_5$ represents $C_1$-$C_5$alkyl, or $C_1$-$C_5$alkyl mono- or poly-substituted by halogen or interrupted by oxygen and/or sulphur, and

X represents the radical of a hydrohalic acid.

8. A pesticidal composition according to claim 7, which contains as active component at least one compound of the formula I or IV in which

$R_1$ represents $C_1$-$C_3$alkyl or cyclopentyl,

$R_2$ represents $C_1$-$C_3$alkyl,

$R_3$ represents hydrogen, and

$R_4$ and $R_5$ represent $C_1$-$C_4$alkyl, and

X represents chlorine.

9. The use of a compound of the formula I

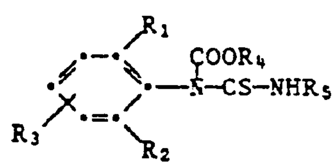

(I)

or of a compound of the formula IV

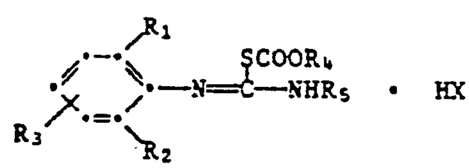

(IV)

in which

$R_1$ and $R_2$ each represents $C_1$-$C_{10}$alkyl, $C_3$-$C_7$cycloalkyl or $C_5$-$C_6$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, and

X represents the radical of a hydrohalic acid, for controlling pests on animals and plants.

10. The use according to claim 9 of a compound of the formula I or IV for controlling insects, arachnids and plant-pathogenic fungi.

11. A method for controlling pests on animals and plants, characterised in that the pests in their various stages of development are brought into contact with a compound of the formula I

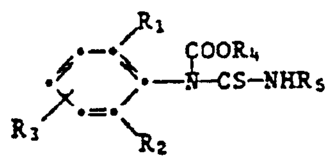

(I)

or with a compound of the formula IV

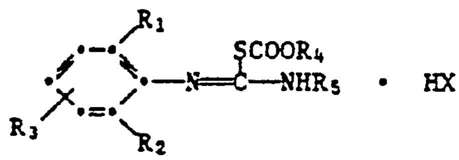

(IV)

in which

$R_1$ and $R_2$ each represents $C_1$-$C_{10}$alkyl, $C_3$-$C_7$cycloalkyl or $C_5$-$C_6$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, and

X represents the radical of a hydrohalic acid.

12. Compounds of the formula IV

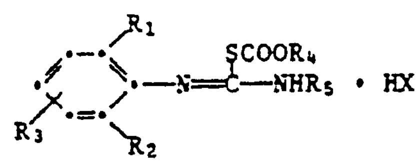

(IV)

in which

$R_1$ and $R_2$ each represents $C_1$-$C_{10}$alkyl, $C_3$-$C_7$cycloalkyl or $C_5$-$C_6$cycloalkenyl,

$R_3$ represents hydrogen, halogen, $C_1$-$C_{10}$alkyl, $C_1$-$C_{10}$alkoxy or $C_1$-$C_{10}$alkylthio,

$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and

$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, and

X represents the radical of a hydrohalic acid.

13. Compounds of the formula IV according to claim 12, in which

$R_1$ represents $C_1$-$C_5$alkyl, cyclopentyl or cyclopentenyl,

$R_2$ represents $C_1$-$C_5$alkyl,

$R_3$ represents hydrogen, halogen, $C_1$-$C_3$alkyl or $C_1$-$C_3$alkoxy,

$R_4$ represents $C_1$-$C_5$alkyl, $C_2$-$C_5$alkenyl or $C_2$-$C_5$alkynyl ; $C_1$-$C_5$alkyl, $C_2$-$C_5$alkenyl, $C_2$-$C_5$alkynyl or phenyl-$C_1$-$C_5$alkyl each mono- or poly-substituted by halogen or interrupted by oxygen and/or sulphur ; or $C_3$-$C_6$cycloalkyl, and

$R_5$ represents $C_1$-$C_5$alkyl, or $C_1$-$C_5$alkyl mono- or poly-substituted by halogen or interrupted by oxygen and/or sulphur, and

X represents the radical of a hydrohalic acid.

14. Compounds of the formula IV according to claim 13, in which

$R_1$ represents $C_1$-$C_3$alkyl or cyclopentyl,
$R_2$ represents $C_1$-$C_3$alkyl,
$R_3$ represents hydrogen, and
$R_4$ and $R_5$ represent $C_1$-$C_4$alkyl, and
X represents chlorine.

15. Compounds according to claim 13 of the formulae

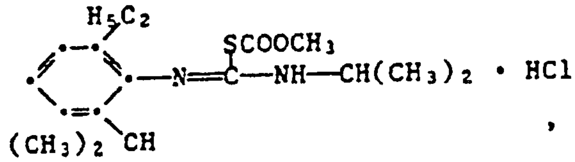

,

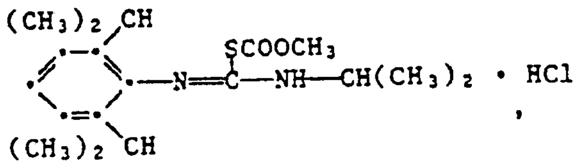

,

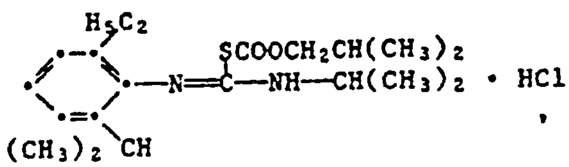

,

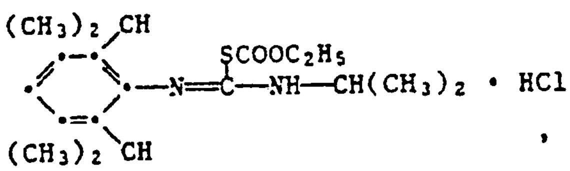

,

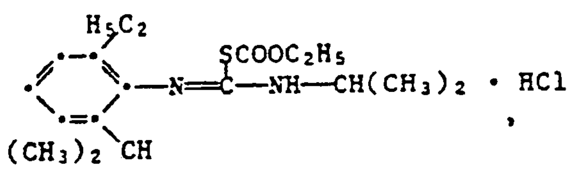

,

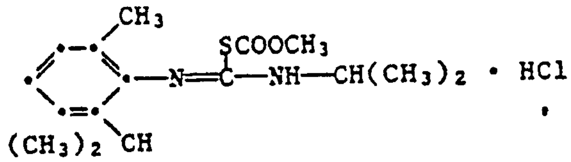

,

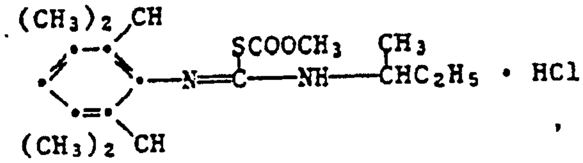

,

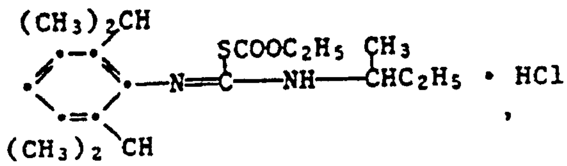

,

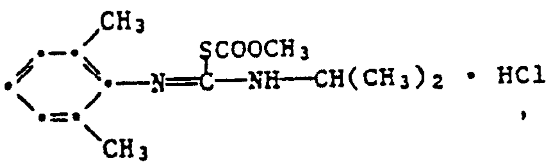

,

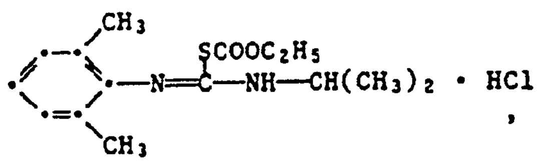

,

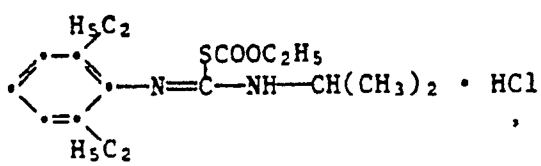

,

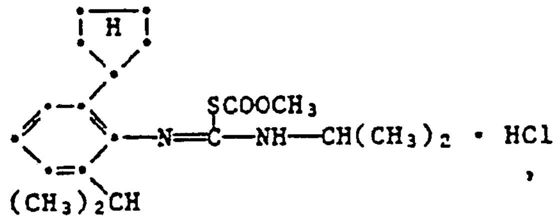

,

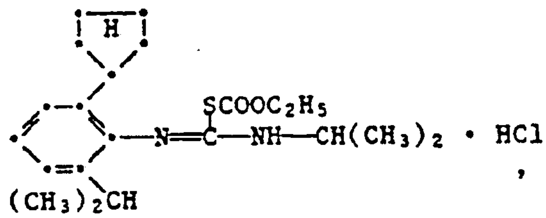

,

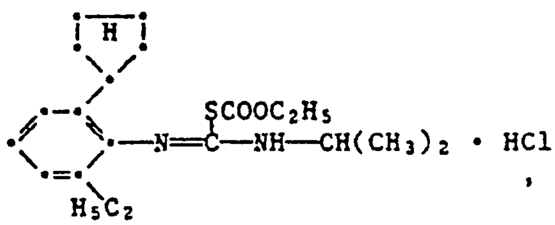

,

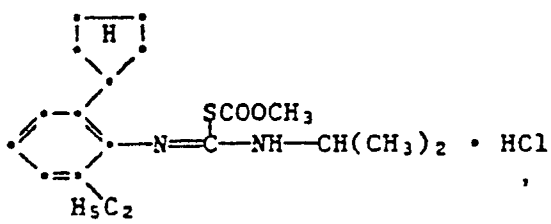

,

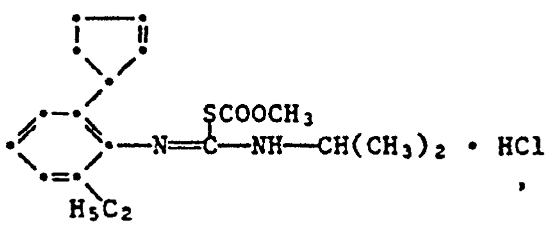

,

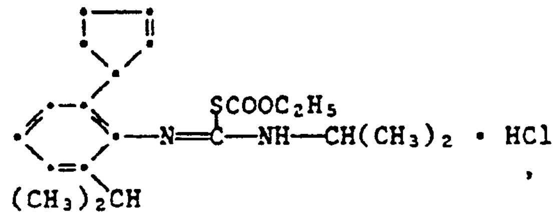

,

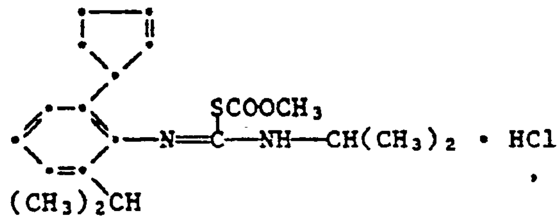

,

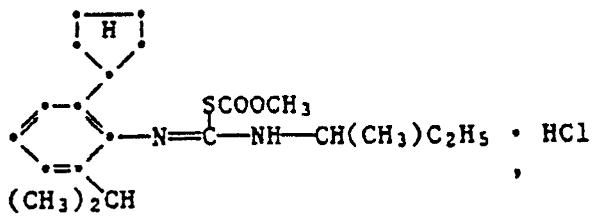

,

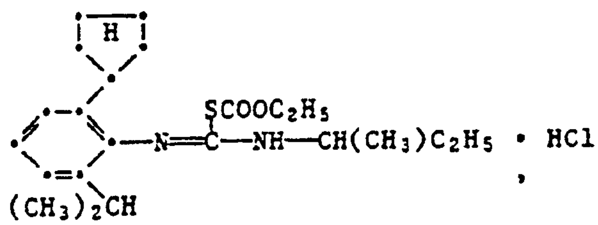

,

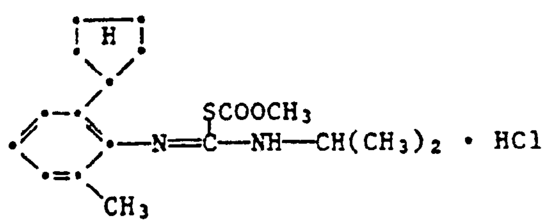

and

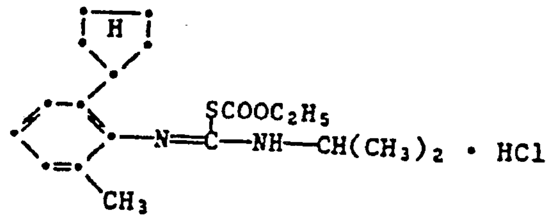

16. A process for the preparation of a compound of the formula IV

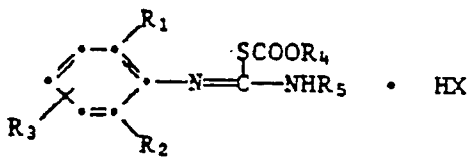 (IV)

in which

$R_1$ and $R_2$ each represents $C_1$-$C_{10}$alkyl, $C_3$-$C_7$cycloalkyl or $C_5$-$C_6$cycloalkenyl,
$R_3$ represents hydrogen, halogen, $C_1$-$C_{10}$alkyl, $C_1$-$C_{10}$alkoxy or $C_1$-$C_{10}$alkylthio,
$R_4$ represents $C_1$-$C_{10}$alkyl, $C_2$-$C_{10}$alkenyl, $C_2$-$C_{10}$alkynyl or phenyl-$C_1$-$C_7$alkyl ; $C_1$-$C_{10}$alkyl, $C_2$-$C_{10}$alkenyl, $C_2$-$C_{10}$alkynyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono- or poly-substituted by halogen or by $C_1$-$C_5$alkyl, and
$R_5$ represents $C_1$-$C_{10}$alkyl ; phenyl-$C_1$-$C_7$alkyl ; $C_1$-$C_{10}$alkyl or phenyl-$C_1$-$C_7$alkyl each mono- or poly-substituted by halogen or interrupted one or more times by oxygen and/or sulphur ; $C_3$-$C_8$cycloalkyl ; or $C_3$-$C_8$cycloalkyl mono-or poly-substituted by halogen or by $C_1$-$C_5$alkyl, and
X represents the radical of a hydrohalic acid, characterised in that a thiourea of the formula II

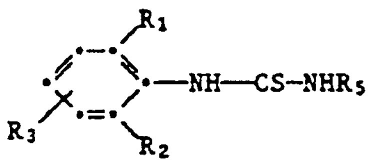 (II)

is reacted with a haloformic acid ester of the formula III

$$XCOOR_4 \quad (III)$$

in a solvent, under normal pressure and at a temperature of from − 20 to + 100°C, $R_1$ to $R_5$ and X having the meanings indicated.

## Revendications

1. Composés de formule I

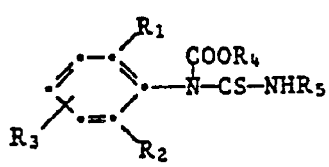 (I),

dans laquelle

$R_1$ et $R_2$ représentent chacun un groupe alkyle en $C_1$-$C_{10}$, cycloalkyle en $C_3$-$C_7$ ou cycloalcényle en $C_5$-$C_6$,

$R_3$ représente l'hydrogène, un halogène, un groupe alkyle en $C_1$-$C_{10}$, alcoxy en $C_1$-$C_{10}$ ou alkylthio en $C_1$-$C_{10}$,

$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

$R_5$ représente un groupe alkyle en $C_1$-$C_{10}$ ; phényl-alkyle en $C_1$-$C_7$, un groupe alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; ou un groupe cycloalkyle en $C_3$-$C_8$ mono- ou poly-substitué par des halogènes ou des groupes alkyle en $C_1$-$C_5$.

2. Composés de formule I selon la revendication 1, pour lesquels

$R_1$ représente un groupe alkyle en $C_1$-$C_5$, cyclopentyle ou cyclopentényle,

$R_2$ représente un groupe alkyle en $C_1$-$C_5$,

$R_3$ représente l'hydrogène, un halogène, un groupe alkyle en $C_1$-$C_3$ ou alcoxy en $C_1$-$C_3$,

$R_4$ représente un groupe alkyle en $C_1$-$C_5$, alcényle en $C_2$-$C_5$, alcynyle en $C_2$-$C_5$ ; un groupe alkyle en $C_1$-$C_5$, alcényle en $C_2$-$C_5$, alcynyle en $C_2$-$C_5$ ou phényl-alkyle en $C_1$-$C_5$ mono- ou poly-substitué par des halogènes ou interrompu une ou plusieurs fois par l'oxygène et/ou le soufre ; ou un groupe cycloalkyle en $C_3$-$C_6$, et

$R_5$ représente un groupe alkyle en $C_1$-$C_5$ ou un groupe alkyle en $C_1$-$C_5$ mono- ou poly-substitué par des halogènes ou interrompu une ou plusieurs fois par l'oxygène et/ou le soufre.

3. Composés de formule I selon la revendication 2, pour lesquels

$R_1$ représente un groupe alkyle en $C_1$-$C_3$ ou cyclopentyle

$R_2$ représente un groupe alkyle en $C_1$-$C_3$,

$R_3$ représente l'hydrogène, et

$R_4$ et $R_5$ représentent des groupes alkyle en $C_1$-$C_4$.

4. Composés selon la revendication 2, de formules

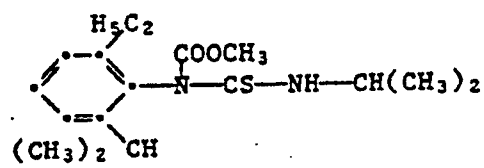
,

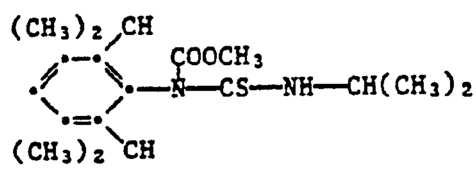
,

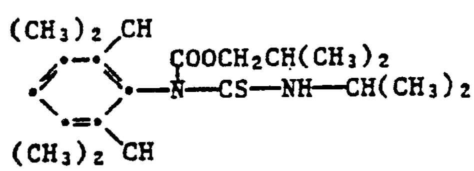
,

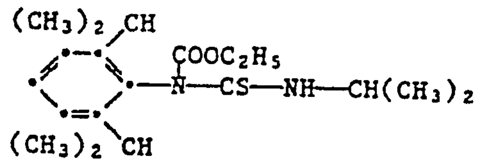

,

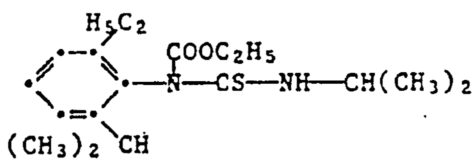

,

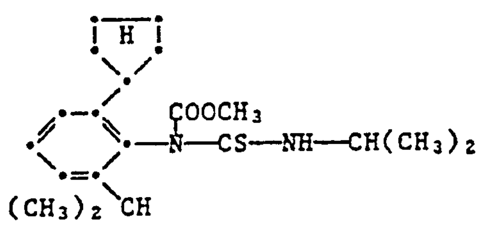

,

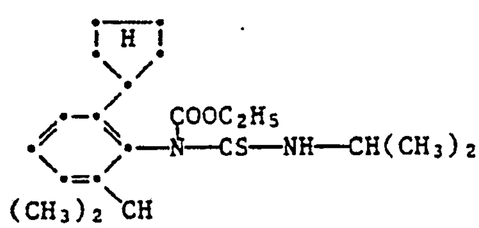

,

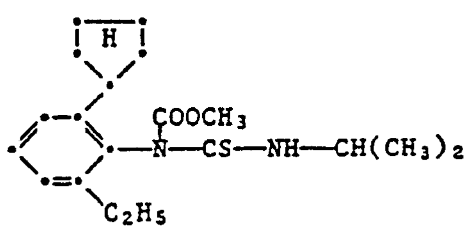

,

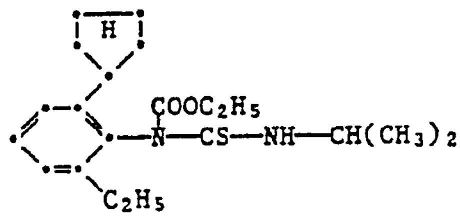

,

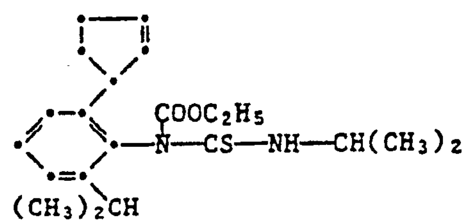

,

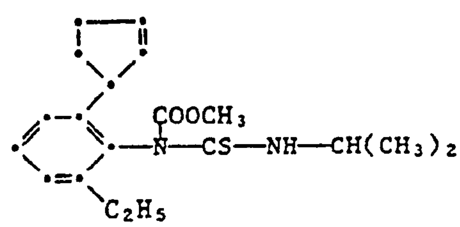

,

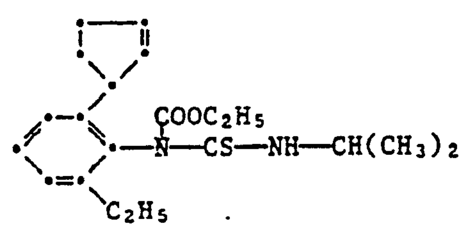

,

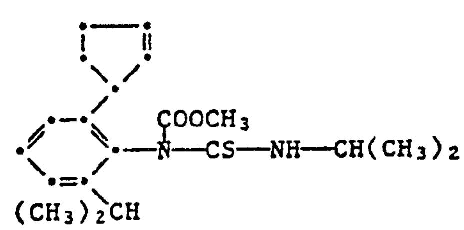

,

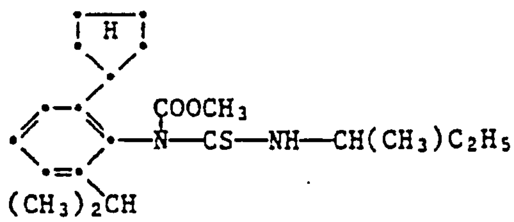

,

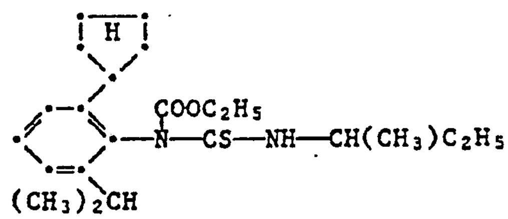

,

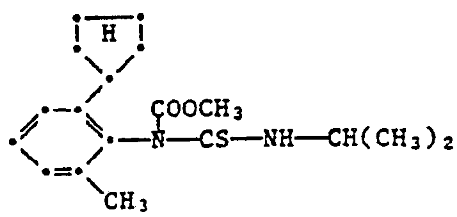

et

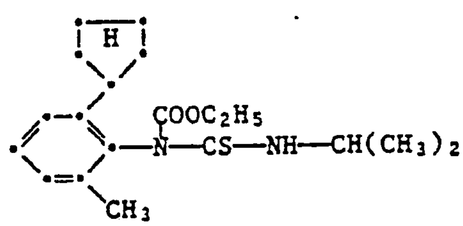

5. Procédé de préparation d'un composé de formule I

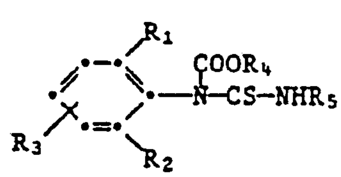

(I),

dans laquelle

$R_1$ et $R_2$ représentent chacun un groupe alkyle en $C_1$-$C_{10}$, cycloalkyle en $C_3$-$C_7$ ou cycloalcényle en $C_5$-$C_6$, $R_3$ représente l'hydrogène, un halogène, un groupe alkyle en $C_1$-$C_{10}$, alcoxy en $C_1$-$C_{10}$ ou alkylthio en $C_1$-$C_{10}$,
$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et
$R_5$ représente un groupe alkyle en $C_1$-$C_{10}$ ; phényl-alkyle en $C_1$-$C_7$ ; alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, caractérisé en ce que :
a) on fait réagir une thiourée de formule II

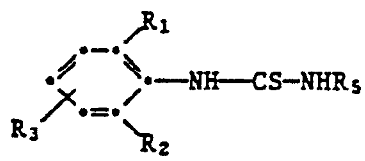

(II)

avec un ester halogénoformique de formule III

$$XCOOR_4 \quad (III)$$

dans un solvant et en présence d'une base, à pression normale, à des températures de − 30 à + 100°C, ou bien
b) on hydrolyse un sel d'une isothiourée de formule IV

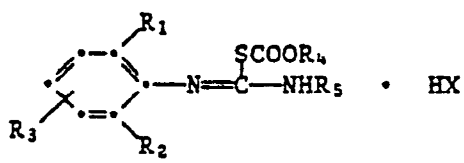

(IV)

à température ambiante, dans une solution aqueuse, les symboles $R_1$ à $R_5$ ayant dans les formules II à IV les significations déjà indiquées et X représente le radical d'un hydracide halogéné.
6. Produit pesticide contenant en tant que composant actif au moins un composé de formule I

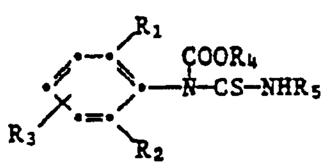

(I)

ou un composé de formule IV

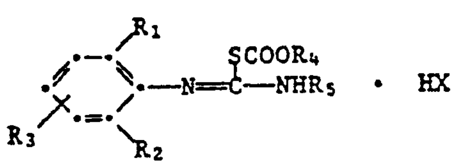

(IV)

dans lesquelles

$R_1$ et $R_2$ représentent chacun un groupe alkyle en $C_1$-$C_{10}$, cycloalkyle en $C_3$-$C_7$ ou cycloalcényle en $C_5$-$C_6$,

$R_3$ représente l'hydrogène, un halogène, un groupe alkyle en $C_1$-$C_{10}$, alcoxy en $C_1$-$C_{10}$ ou alkylthio en $C_1$-$C_{10}$,

$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

$R_5$ représente un groupe alkyle en $C_1$-$C_{10}$ ; phényl-alkyle en $C_1$-$C_7$ ; alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

X représente le radical d'un hydracide halogéné, avec des véhicules et/ou additifs appropriés.

7. Produit pesticide selon la revendication 6, contenant en tant que composé actif au moins un composé de formule I ou IV dans laquelle

$R_1$ représente un groupe alkyle en $C_1$-$C_5$, cyclopentyle ou cyclopentényle,

$R_2$ représente un groupe alkyle en $C_1$-$C_5$,

$R_3$ représente l'hydrogène, un halogène, un groupe alkyle en $C_1$-$C_3$ ou alcoxy en $C_1$-$C_3$,

$R_4$ représente un groupe alkyle en $C_1$-$C_5$, alcényle en $C_2$-$C_5$, alcynyle en $C_2$-$C_5$ ; un groupe alkyle en $C_1$-$C_5$, alcényle en $C_2$-$C_5$, alcynyle en $C_2$-$C_5$ ou phényl-alkyle en $C_1$-$C_5$ mono- ou poly-substitué par des halogènes ou interrompu une ou plusieurs fois par l'oxygène et/ou le soufre ; ou un groupe cycloalkyle en $C_3$-$C_6$ et

$R_5$ représente un groupe alkyle en $C_1$-$C_5$ ou un groupe alkyle en $C_1$-$C_5$ mono- ou poly-substitué par des halogènes ou interrompu une ou plusieurs fois par l'oxygène et/ou le soufre, et

X représente le radical d'un hydracide halogéné.

8. Produit pesticide selon la revendication 7, contenant en tant que composant actif au moins un composé de formule I ou IV dans laquelle

$R_1$ représente un groupe alkyle en $C_1$-$C_3$ ou cyclopentyle,

$R_2$ représente un groupe alkyle en $C_1$-$C_3$,

$R_3$ représente l'hydrogène, et

$R_4$ et $R_5$ représentent des groupes alkyle en $C_1$-$C_4$, et

X représente le chlore.

9. Utilisation d'un composé de formule I

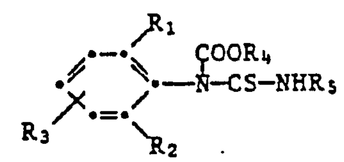

(I)

ou d'un composé de formule IV

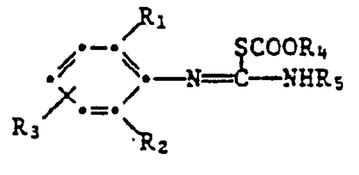

• HX (IV),

dans lesquelles

$R_1$ et $R_2$ représentent chacun un groupe alkyle en $C_1$-$C_{10}$, cycloalkyle en $C_3$-$C_7$ ou cycloalcényle en $C_5$-$C_6$,

$R_3$ représente l'hydrogène, un halogène, un groupe alkyle en $C_1$-$C_{10}$, alcoxy en $C_1$-$C_{10}$ ou alkylthio en $C_1$-

$C_{10}$,

$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

$R_5$ représente un groupe alkyle en $C_1$-$C_{10}$ ; phényl-alkyle en $C_1$-$C_7$ ; un groupe alkyle en $C_1$-$C_{10}$ ou phényl-alkyle en $C_1$-$C_7$ mono- ou poly-substitué par des halogenes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes : ou des groupes alkyle en $C_1$-$C_5$, et

X représente le radical d'un hydracide halogéné, pour la lutte contre les parasites des animaux et des végétaux.

10. Utilisation selon la revendication 9 d'un composé de formule I ou IV pour la lutte contre les insectes, les arachnides et les mycètes phytopathogènes.

11. Procédé pour combattre les parasites des animaux et des végétaux, caractérisé en ce que l'on met en contact les parasites, dans leurs divers stades de développement, avec un composé de formule I

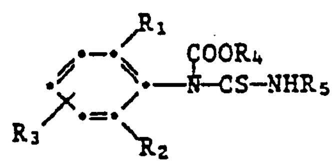

(I)

ou avec un composé de formule IV

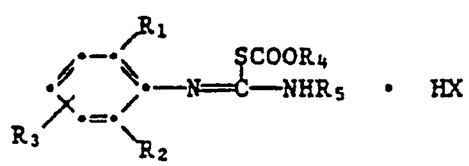

(IV),

dans lesquelles

$R_1$ et $R_2$ représentent chacun un groupe alkyle en $C_1$-$C_{10}$, cycloalkyle en $C_3$-$C_7$ ou cycloalcényle en $C_5$-$C_6$,

$R_3$ représente l'hydrogène, un halogène, un groupe alkyle en $C_1$-$C_{10}$, alcoxy en $C_1$-$C_{10}$ ou alkylthio en $C_1$-$C_{10}$,

$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; un groupe cycloalkyle en $C_3$-$C_8$ mono- ou poly-substitué par des halogènes ou des groupes alkyle en $C_1$-$C_5$, et

$R_5$ représente un groupe alkyle en $C_1$-$C_{10}$ ; phényl-alkyle en $C_1$-$C_7$ ; un groupe alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

X représente le radical d'un hydracide halogéné.

12. Composés de formule IV

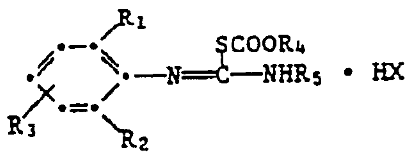

(IV),

dans laquelle

$R_1$ et $R_2$ représentent chacun un groupe alkyle en $C_1$-$C_{10}$, cycloalkyle en $C_3$-$C_7$ ou cycloalcényle en $C_5$-$C_6$,

$R_3$ représente l'hydrogène, un halogène, un groupe alkyle en $C_1$-$C_{10}$, alcoxy en $C_1$-$C_{10}$ ou alkylthio en $C_1$-$C_{10}$,

$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

$R_5$ représente un groupe alkyle en $C_1$-$C_{10}$ ; phényl-alkyle en $C_1$-$C_7$ ; un groupe alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et

X représente le radical d'un hydracide halogéné.

13. Composé de formule IV selon la revendication 12, dans laquelle

$R_1$ représente un groupe alkyle en $C_1$-$C_5$, cyclopentyle ou cyclopentényle,

$R_2$ représente un groupe alkyle en $C_1$-$C_5$,

$R_3$ représente l'hydrogène, un halogène, un groupe alkyle en $C_1$-$C_3$ ou alcoxy en $C_1$-$C_3$,

$R_4$ représente un groupe alkyle en $C_1$-$C_5$, alcényle en $C_2$-$C_5$, alcynyle en $C_2$-$C_5$ ; un groupe alkyle en $C_1$-$C_5$, alcényle en $C_2$-$C_5$, alcynyle en $C_2$-$C_5$ ou phényl-alkyle en $C_1$-$C_5$ mono- ou poly-substitué par des halogènes ou interrompu une ou plusieurs fois par l'oxygène et/ou le soufre ; ou un groupe cycloalkyle en $C_3$-$C_6$, et

$R_5$ représente un groupe alkyle en $C_1$-$C_5$ ou un groupe alkyle en $C_1$-$C_5$ mono- ou poly-substitué par des halogènes ou interrompu une ou plusieurs fois par l'oxygène et/ou le soufre, et

X représente le radical d'un hydracide halogéné.

14. Composés de formule IV selon la revendication 13, dans laquelle

$R_1$ représente un groupe alkyle en $C_1$-$C_3$ ou cyclopentyle,

$R_2$ représente un groupe alkyle en $C_1$-$C_3$,

$R_3$ représente l'hydrogène, et

$R_4$ et $R_5$ représentent des groupes alkyle en $C_1$-$C_4$, et

X représente le chlore.

15. Composés selon la revendication 13, de formules

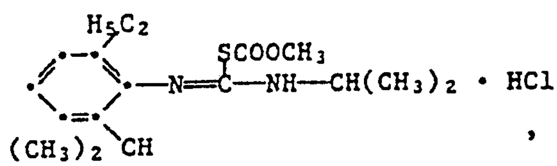

,

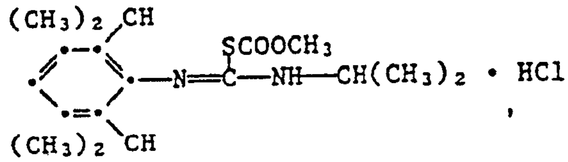

,

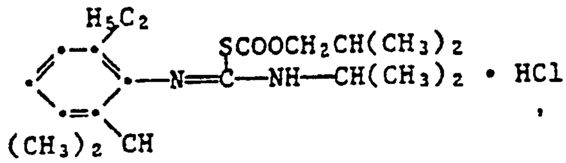

,

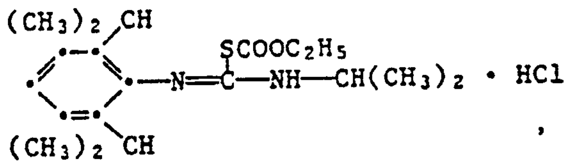

,

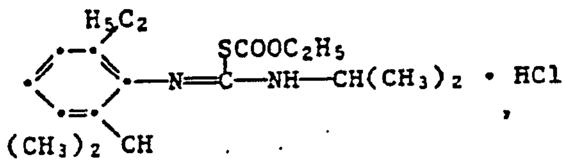

,

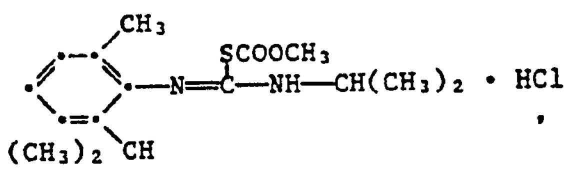

,

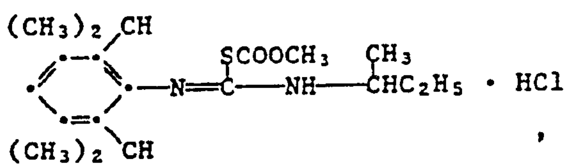

,

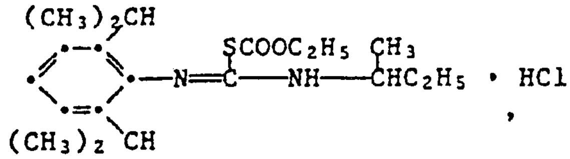

,

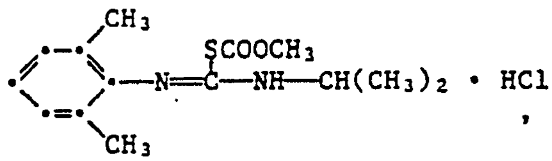

,

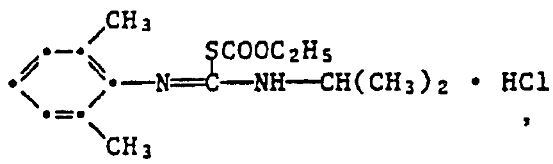

,

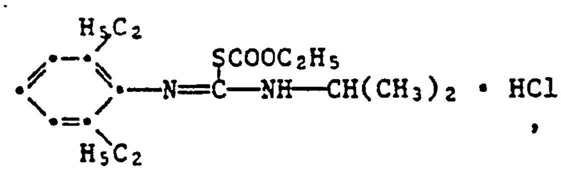

,

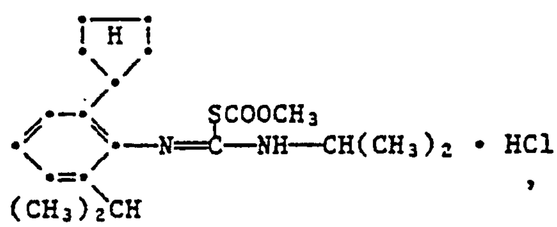

,

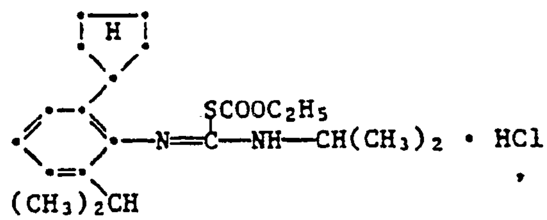

,

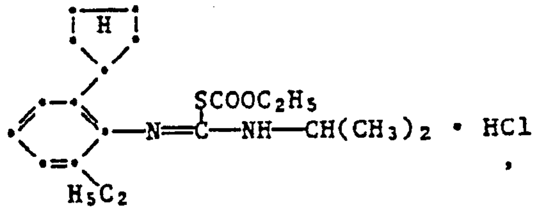

,

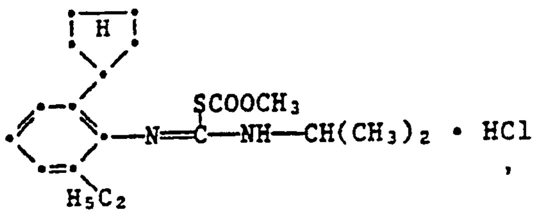

,

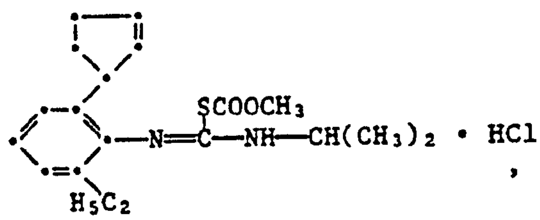

,

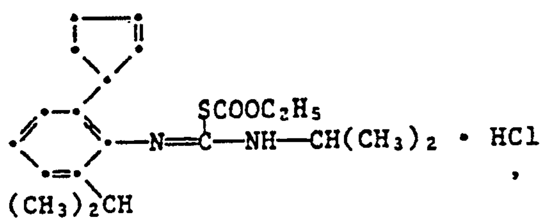

,

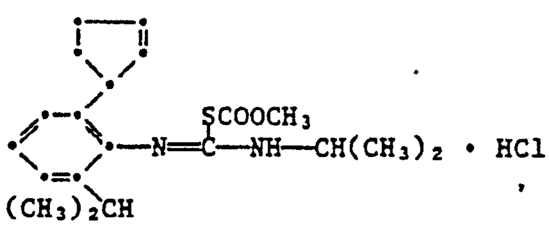

,

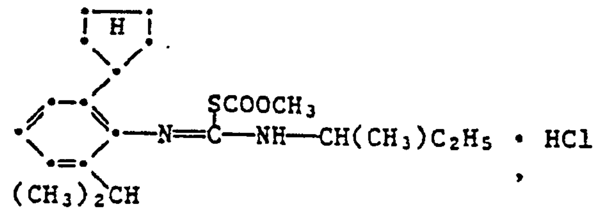

,

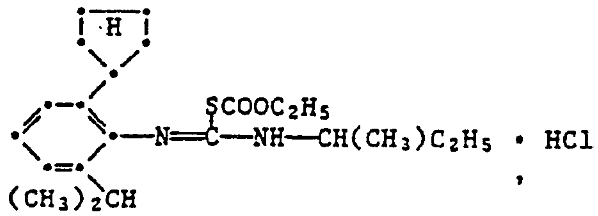

,

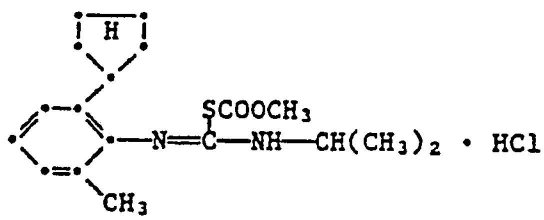

et

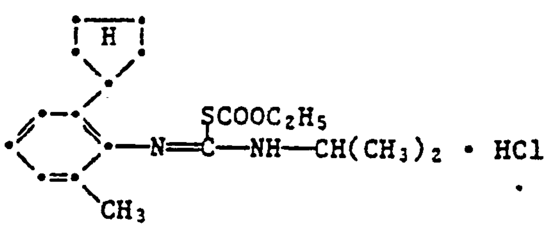

.

16. Procédé de préparation d'un composé de formule IV

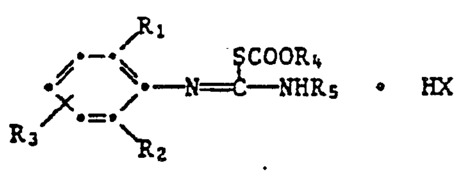

(IV),

dans laquelle

$R_1$ et $R_2$ représentent chacun un groupe alkyle en $C_1$-$C_{10}$, cycloalkyle en $C_3$-$C_7$ ou cycloalcényle en $C_5$-$C_6$,
$R_3$ représente l'hydrogène, un halogène, un groupe alkyle en $C_1$-$C_{10}$, alcoxy en $C_1$-$C_{10}$ ou alkylthio en $C_1$-

$C_{10}$,
$R_4$ représente un groupe alkyle en $C_1$-$C_{10}$, alcényle en $C_2$-$C_{10}$, alcynyle en $C_2$-$C_{10}$, phényl-alkyle en $C_1$-$C_7$; un groupe alkyle en $C_1$-$C_{10}$, alcényle en $C_2$-$C_{10}$, alcynyle en $C_2$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et
$R_5$ représente un groupe alkyle en $C_1$-$C_{10}$ ; phényl-alkyle en $C_1$-$C_7$ ; alkyle en $C_1$-$C_{10}$ ou phényl-alkyle en $C_1$-$C_7$ mono- ou poly-substitué par des halogènes ou interrompu une ou plusieurs fois par l'oxygène et/ou le soufre ; un groupe cycloalkyle en $C_3$-$C_8$ ; ou un groupe cycloalkyle en $C_3$-$C_8$ mono- ou poly-substitué par des halogènes ou des groupes alkyle en $C_1$-$C_5$, et
X représente le radical d'un hydracide halogéné, caractérisé en ce que l'on fait réagir une thiourée de formule II

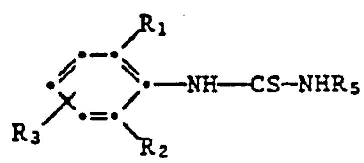

(II)

dans un solvant, à pression normale, à une température de – 20 à + 100°C, avec un ester halogénoformique de formule III

$$XCOOR_4 \quad (III)$$

$R_1$ $R_5$ et X ayant les significations indiquées ci-dessus.